# EUROPEAN PATENT APPLICATION

(11) **EP 3 919 058 A1**
(43) Date of publication of application: **08.12.2021**
(21) Application number: 20748834.7
(22) Date of filing: 22.01.2020
(51) Int. Cl.: A61K 31/519, A61K 31/4196, C07D 471/04, A61P 35/00

(54) **USE OF COMPOSITION CONTAINING CDK4/6 INHIBITOR IN COMBINATION WITH ANASTROZOLE IN PREPARATION OF MEDICAMENT FOR TREATING TUMOR DISEASES**

(30) Priority: 30.01.2019 CN 201910089738; 25.04.2019 CN 201910339438; 14.06.2019 CN 201910516489; 10.07.2019 CN 201910620303; 24.09.2019 CN 201910907616
(71) Applicant: Jiangsu Hengrui Medicine Co., Ltd., Lianyungang, Jiangsu 222047 (CN)
(72) Inventor: ZHU, Xiaoyu, Lianyungang, Jiangsu 222047 (CN); LI, Guorong, Lianyungang, Jiangsu 222047 (CN); ZOU, Jianjun, Lianyungang, Jiangsu 222047 (CN)
(74) Representative: dompatent von Kreisler Selting Werner - Partnerschaft von Patent- und Rechtsanwälten mbB
(86) International application number: PCT/CN2020/073815
(87) International publication number: WO 2020/156446

(57) **Abstract**

The present disclosure relates to use of a composition containing a CDK4/6 inhibitor in combination with anastrozole in preparation of a medicament for treating tumor diseases. Specifically, the CDK4/6 inhibitor involved in the application is a compound represented by formula(I) or a pharmaceutically acceptable salt thereof.

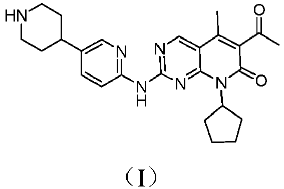

## Description

The present application claims the priorities of Chinese Patent Application No. CN201910089738.2 filed on January 30, 2019, Chinese Patent Application No. CN201910339438.5 filed on April 25, 2019, Chinese Patent Application No. CN201910516489.0 filed on June 14, Chinese Patent Application No. CN201910620303.6 filed on July 10, 2019 and Chinese Patent Application No. CN201910907616.X filed on September24, 2019. The contents of which are incorporated herein by reference in their entirety.

### Technical Field

A use of a composition containing a CDK4/6 inhibitor in combination with anastrozole in the preparation of a medicament for treating tumors.

### Background arts

Breast cancer is one of the most common malignant tumors in women, with approximately 1.3 million new cases occurring worldwide each year. In China, the incidence of breast cancer accounts for 7% to 10% of all malignant tumors in the whole body, and accounts for approximately 18% of all tumors of the female;at present, the number of patients in China has exceeded 500,000, and its incidence has increased rapidly, ranking first in the incidence spectrum of tumors of the female in some big cities, and nearly 50% of patients have recurrence and metastasis after treatment. In recent years, with the deepening of tumor molecular biology research, molecular targeted therapy has been widely used in the treatment of breast cancer and achieved remarkable curative effect. It has become a new treatment mode after the three traditional modes of surgery, radiotherapy and chemotherapy, and is also a hot spot in the field of breast cancer treatment.

Cyclin-dependent kinase (CDK) is a type of serine/threonine kinase, which forms a dimer with the corresponding cyclin, and then phosphorylates the downstream protein molecules, thus promoting the orderly progress of each phase of the cell cycle and realizing cell growth and proliferation. At present, a variety of CDK4/6 selective inhibitors have been listed in the clinical trial stage or approved for marketing, including Palbociclib in Pfizer, Ribociclib in Novartis and Abemaciclib in Lilly, etc.

PCT application WO2014183520 disclosed a CDK4/6 inhibitor with chemical name of 6-acetyl-8-cyclopentyl-5-methyl-2-((5-(piperidin-4-yl)pyridin-2-yl)amino)pyrido[2,3-d]pyrimi din-7(8H)-one as shown in formula (I),which has significant inhibitory activity and high selectivity of CDK4/6.

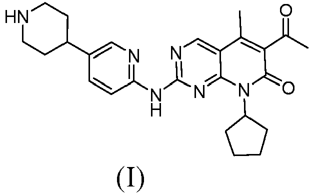

PCT application WO2016124067A disclosed a hydroxyethyl sulfonate of the compound represented by formula (I) above and a preparation method thereof. The prior art CN107137408A also investigated the combination of the compound represented by formula (I) and the pharmaceutically acceptable salt thereof with aromatic kinase inhibitors. A pharmacokinetic research of letrozole and anastrozole as a single agent showed that letrozole (2.5 mg once a day) is a more effective systemic aromatization and plasma estrogen level inhibitor than anastrozole (1 mg daily) in postmenopausal women with metastatic breast cancer (PMID : 11821457). Another open randomized trial comparingletrozoleandanastrozolein second-line endocrine therapy foradvanced breast cancer concluded that, in support of the prior description of letrozole's higher aromatase inhibitory activity, advanced breast cancer responded more strongly to letrozole than to anastrozole as second-line hormonal therapy (PMID: 14556923).

European Journal of Cancer 39 (2003) 2318―2327 relates to an open randomized trial of second-line endocrine therapy for advanced breast cancer, specifically the comparison between aromatase inhibitors letrozole and anastrozole, the part of the abstract disclosed that letrozole is significantly superior to anastrozole in terms of total remission rate (ORR).

### Content of the present invention

The present disclosure provides a use of a pharmaceutical composition containing a compound represented by formula (I) or a pharmaceutically acceptable salt thereof in combination with anastrozole in the preparation of a medicament for treating tumor diseases,

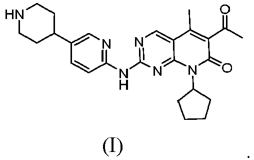

The tumor diseases described in this disclosure are selected from one or more of sarcoma, lymphoma, lung cancer, bronchial cancer, prostate cancer, pancreatic cancer, gastrointestinal cancer, colon cancer, rectal cancer, colorectal adenoma, thyroid cancer, liver cancer, intrahepatic cholangiocarcinoma, hepatocellular carcinoma, adrenal cancer, gastric cancer, gastric tumor, glioma, glioblastoma, endometrial cancer, melanoma, kidney cancer, renal pelvis cancer, bladder cancer, uterine body cancer, cervical cancer, vaginal cancer, ovarian cancer, multiple myeloma, esophageal cancer, leukemia, acute myeloid leukemia, chronic myeloid leukemia, lymphocytic leukemia, myeloid leukemia, brain tumor, brain cancer, oral and pharyngeal cancer, laryngeal cancer, small intestine cancer, non-Hodgkin's lymphoma, melanoma, colonic villous adenoma, neoplasm, epithelial cancer, breast cancer, basal cell carcinoma, squamous cell carcinoma, actinic keratosis, tumor disease, neck or head tumor, primary thrombocythemia, myeloid metaplasticmyelofibrosis, and macroglobulinemia, preferably breast cancer.

The breast cancer described in the present disclosure is preferably hormone receptor positive (HR+) and/or human epidermal growth factor receptor 2 negative (HER2-); more preferably locally advanced or metastatic breast cancer of the above phenotype.

In the present disclosure, the pharmaceutically acceptable salt of the compound represented by formula (I) is selected from one or more of hydrochloride, phosphate, hydrogen phosphate, sulfate, hydrogen sulfate, sulfite, acetate, oxalate, malonate, valerate, glutamate, oleate, palmitate, stearate, laurate, borate, *p*-toluenesulfonate, methanesulfonate, hydroxyethyl sulfonate, maleate, malate, tartarate, benzoate, dihydroxynaphthalenate, salicylate, vanillate, lentilate, succinate, gluconate, lactonate and lauryl sulfonate, preferably hydroxyethyl sulfonate, the structure of which is represented by formula (II),

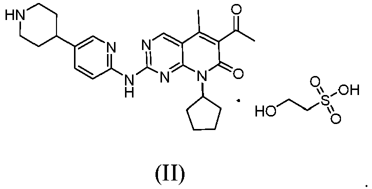

In some embodiments of the present disclosure, the amount of the compound represented by formula (I) or the pharmaceutically acceptable salt thereof in the pharmaceutical composition containing the compound represented by formula (I) or the pharmaceutically acceptable salt thereof administered daily is 1-500 mg, preferably 50-200 mg, more preferably 100-150 mg.

In some preferable embodiments of the present disclosure, the amount of the compound represented by formula (I) or the pharmaceutically acceptable salt thereof in the pharmaceutical composition containing the compound represented by formula (I) or the pharmaceutically acceptable salt thereof administered daily is 50 mg, 75 mg, 100 mg, 125 mg, 150 mg, 175 mg, preferably 100 mg, 125 mg or 150mg.

In the preferable embodiments of the present disclosure, the administration frequency of the pharmaceutical composition containing the compound represented by formula (I) or the pharmaceutically acceptable salt thereof is once a day, twice a day, preferably once a day.

In some embodiments of the present disclosure, the daily dose of anastrozole is selected from 0.1-50 mg, preferably 1-25 mg, and most preferably 1-10 mg.

In the preferable embodiments of the present disclosure, the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg.

In the preferable embodiments of the present disclosure, the administration frequency of anastrozole is once a day, twice a day, preferably once a day.

In the preferable embodiments of the present disclosure, the amount of the compound represented by formula (I) or the pharmaceutically acceptable salt thereof in the pharmaceutical composition containing the compound represented by formula (I) or the pharmaceutically acceptable salt thereof administered daily is 50 mg, 75 mg, 100 mg, 125 mg, 150 mg, 175 mg, and the administration frequency of the pharmaceutical composition containing the compound represented by formula (I) or the pharmaceutically acceptable salt thereof is once a day, twice a day, preferably once a day, the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg, and the administration frequency of anastrozole is once a day, twice a day, preferably once a day.

In the preferable embodiments of the present disclosure, the amount of the compound represented by formula (I) or the pharmaceutically acceptable salt thereof in the pharmaceutical composition containing the compound represented by formula (I) or the pharmaceutically acceptable salt thereof administered daily is 100 mg, 125 mg, 150 mg, and the administration frequency of the pharmaceutical composition containing the compound represented by formula (I) or the pharmaceutically acceptable salt thereof is once a day, the daily dose of anastrozole is 1.0 mg, and the administration frequency of anastrozole is once a day.

In a embodiment of the present disclosure, the amount of the compound represented by formula (I) or the pharmaceutically acceptable salt thereof in the pharmaceutical composition containing the compound represented by formula (I) or the pharmaceutically acceptable salt thereof administered daily is 100 mg, and the administration frequency of the pharmaceutical composition containing the compound represented by formula (I) or the pharmaceutically acceptable salt thereof is once a day, the daily dose of anastrozole is 1.0 mg, and the administration frequency of anastrozole is once a day.

In a embodiment of the present disclosure, the amount of the compound represented by formula (I) or the pharmaceutically acceptable salt thereof in the pharmaceutical composition containing the compound represented by formula (I) or the pharmaceutically acceptable salt thereof administered daily is 125 mg, and the administration frequency of the pharmaceutical composition containing the compound represented by formula (I) or the pharmaceutically acceptable salt thereof is once a day, the daily dose of anastrozole is 1.0 mg, and the administration frequency of anastrozole is once a day.

In a embodiment of the present disclosure, the amount of the compound represented by formula (I) or the pharmaceutically acceptable salt thereof in the pharmaceutical composition containing the compound represented by formula (I) or the pharmaceutically acceptable salt thereof administered daily is 150 mg, and the administration frequency of the pharmaceutical composition containing the compound represented by formula (I) or the pharmaceutically acceptable salt thereof is once a day, the daily dose of anastrozole is 1.0 mg, and the administration frequency of anastrozole is once a day.

The pharmaceutical composition containing the compound represented by formula (I) or the pharmaceutically acceptable salt thereof in the present disclosure may further contain at least one pharmaceutically acceptable excipient, the excipient is selected from diluent, binder, disintegrant and lubricant.

The content of the diluent in the pharmaceutical composition containing the compound represented by formula (I) or the pharmaceutically acceptable salt thereof in the present disclosure may be 1%-99% by weight, preferably 5%-95% by weight relative to the total weight of the pharmaceutical composition.

The content of the lubricant in the pharmaceutical composition containing the compound represented by formula (I) or the pharmaceutically acceptable salt thereof in the present disclosure may be 0.01%-25% by weight, preferably 0.1%-10% by weight relative to the total weight of the pharmaceutical composition.

Anastrozole in the present disclosure can be administered in the form of a pharmaceutical composition, and specifically, anastrozole is administered in the form of a pharmaceutical composition suitable for enteral administration or parenteral administration, such as tablets, capsules, suppositories, ampoules, injection solutions or injection suspensions. Anastrozole can be administered, for example, in a commercially available form (e.g., ARIMlDEX○, R).

In the preferable embodiments of the present disclosure, the pharmaceutical composition containing the compound represented by formula (I) or the pharmaceutically acceptable salt thereof and anastrozole are both administered orally.

In some embodiments, the present disclosure provides a use of the pharmaceutical composition containing the compound represented by formula (I) or the pharmaceutically acceptable salt thereof in combination with anastrozole in the preparation of a medicament for treating tumors, wherein anastrozole increases the AUC of the compound represented by formula (I) or the pharmaceutically acceptable salt thereof in patients.

In some embodiments, the present disclosure provides a use of the pharmaceutical composition containing the compound represented by formula (I) or the pharmaceutically acceptable salt thereof in combination with anastrozole in the preparation of a medicament for treating tumors, wherein anastrozole reduces the incidence of adverse reactions in patients. Wherein, the adverse reaction is preferably hematological toxicity, and the hematological toxicity may be selected from neutropenia, leukopenia and/or thrombocytopenia, preferably grade 3, 4 neutropenia and/or grade 3, 4 leukopenia.

In the preferable embodiments, the patient with tumor diseases in the present disclosure is a human.

On the other hand, the present disclosure provides a method for increasing AUC of the compound represented by formula (I) or the pharmaceutically acceptable salt thereof in a patient, which comprises administering a therapeutically effective amount of the compound represented by formula (I) or the pharmaceutically acceptable salt thereof and a therapeutically effective amount of anastrozole to the patient, and the patient is preferably a human.

In the present disclosure, "increasing AUC of the compound represented by formula (I) or the pharmaceutically acceptable salt thereof in a patient" refers to increasing relative to AUC produced in the patient by administering an equivalent dose of the compound represented by formula (I) or the pharmaceutically available salt thereof alone, and/or AUC produced in the patient by administering an equivalent dose of the compound represented by formula (I) or the pharmaceutically acceptable salt thereof in combination withletrozole. "Reducing the incidence of adverse reactions in a patient" also refers to that of the compound represented by formula (I) in combination with anastrozole relative to that of an equivalent dose of the compound represented by formula (I) administered alone and/or relative to that of an equivalent dose of the compound represented by formula (I) or the pharmaceutically acceptable salt in combination with letrozole.

In some embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof alone, the AUC produced in the patient is increased by more than 18%, optionally, the AUC is increased by 20%-150%, optionally, the AUC is increased by 25%-100%; optionally, the AUC is increased by 30%-85%; optionally, the AUC is increased by 35%-80%; optionally, the AUC is increased by 40%-80%; preferably, the AUC is increased by 45%-80%; preferably, the AUC is increased by 50%-80%, preferably, the AUC is increased by 55%-80%; preferably, the AUC is increased by 60%-80%; specifically, the AUC of the compound represented by formula (I) or the pharmaceutically available salt thereof is increases by about 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%.

In some embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof in combination with letrozole, the AUC ratio of the compound represented by formula (I) or the pharmaceutically available salt thereof in the patient is more than 1:1, optionally, the AUC ratio is selected from 1 to 10:1; optionally, the AUC ratio is selected from 1 to 5:1; optionally, the AUC ratio is selected from 1 to 4:1; optionally, the AUC ratio is selected from 1 to 3:1; preferably, the AUC ratio is selected from 1 to 2:1; preferably, the AUC ratio is selected from 1 to 1.5:1; specifically, the AUC ratio may be 1.01 :1, 1.02 :1, 1.03 :1, 1.04 :1, 1.05 :1, 1.06 :1, 1.07 :1, 1.08 :1, 1.09 :1, 1.10 :1, 1.11 :1, 1.12 :1, 1.13 :1, 1.14 :1, 1.15 :1, 1.16 :1, 1.17 :1, 1.18 :1, 1.19 :1, 1.20 :1, 1.21 :1, 1.22 :1, 1.23 :1, 1.24 :1, 1.25 :1, 1.26 :1, 1.27 :1, 1.28 :1, 1.29 :1, 1.30 :1, 1.31 :1, 1.32 :1, 1.33 :1, 1.34 :1, 1.35 :1, 1.36 :1, 1.37 :1, 1.38 :1, 1.39 :1, 1.40 :1, 1.41 :1, 1.42 :1, 1.43 :1, 1.44 :1, 1.45 :1, 1.46 :1, 1.47 :1, 1.48 :1, 1.49 :1, 1.50 :1, 1.51 :1, 1.52 :1, 1.53 :1, 1.54 :1, 1.55 :1, 1.56 :1, 1.57 :1, 1.58 :1, 1.59 :1, 1.60 :1, 1.61 :1, 1.62 :1, 1.63 :1, 1.64 :1, 1.65 :1, 1.66 :1, 1.67 :1, 1.68 :1, 1.69 :1, 1.70 :1, 1.71 :1, 1.72 :1, 1.73 :1, 1.74 :1, 1.75 :1, 1.76 :1, 1.77 :1, 1.78 :1, 1.79 :1, 1.80 :1, 1.81 :1, 1.82 :1, 1.83 :1, 1.84 :1, 1.85 :1, 1.86 :1, 1.87 :1, 1.88 :1, 1.89 :1, 1.90 :1, 1.91 :1, 1.92 :1, 1.93 :1, 1.94 :1, 1.95 :1, 1.96 :1, 1.97 :1, 1.98 :1, 1.99 :1, 2.00:1.

In some embodiments, by administering to the patient an effective amount of the compound represented by formula (I) or the pharmaceutically acceptable salt thereof and an effective amount of anastrozole, the compound represented by formula (I) or the pharmaceutically acceptable salt thereof provides an AUC of 2800-8000 ng^{∗}h/mL in a patient; optionally, the compound represented by formula (I) or the pharmaceutically acceptable salt thereof provides an AUC of 2800-7800 ng^{∗}h/mL in a patient; optionally, the compound represented by formula (I) or the pharmaceutically acceptable salt thereof provides an AUC of 3000-7600 ng^{∗}h/mL in a patient; optionally, the compound represented by formula (I) or the pharmaceutically acceptable salt thereof provides an AUC of 3000-7400 ng^{∗}h/mL in a patient; optionally, the compound represented by formula (I) or the pharmaceutically acceptable salt thereof provides an AUC of 3000-7200 ng^{∗}h/mL in a patient; optionally, the compound represented by formula (I) or the pharmaceutically acceptable salt thereof provides an AUC of 3000-7000 ng^{∗}h/mL in a patient; optionally, the compound represented by formula (I) or the pharmaceutically acceptable salt thereof provides an AUC of 3000-6800 ng^{∗}h/mL in a patient; optionally, the compound represented by formula (I) or the pharmaceutically acceptable salt thereof provides an AUC of 3000-6600 ng^{∗}h/mL in a patient; optionally, the compound represented by formula (I) or the pharmaceutically acceptable salt thereof provides an AUC of 3000-6400 ng^{∗}h/mL in a patient; optionally, the compound represented by formula (I) or the pharmaceutically acceptable salt thereof provides an AUC of 3000-6200 ng^{∗}h/mL in a patient; optionally, the compound represented by formula (I) or the pharmaceutically acceptable salt thereof provides an AUC of 3000-6000 ng^{∗}h/mL in a patient; optionally, the compound represented by formula (I) or the pharmaceutically acceptable salt thereof provides an AUC of 3000-5800 ng^{∗}h/mL in a patient; optionally, the compound represented by formula (I) or the pharmaceutically acceptable salt thereof provides an AUC of 3000-5600 ng^{∗}h/mL in a patient; optionally, the compound represented by formula (I) or the pharmaceutically acceptable salt thereof provides an AUC of 3000-5400 ng^{∗}h/mL in a patient; optionally, the compound represented by formula (I) or the pharmaceutically acceptable salt thereof provides an AUC of 3000-5200 ng^{∗}h/mL in a patient; optionally, the compound represented by formula (I) or the pharmaceutically acceptable salt thereof provides an AUC of 3000-5000 ng^{∗}h/mL in a patient; preferably, the compound represented by formula (I) or the pharmaceutically acceptable salt thereof provides an AUC of 3000-4800 ng^{∗}h/mL in a patient; preferably, the compound represented by formula (I) or the pharmaceutically acceptable salt thereof provides an AUC of 3200-4600 ng^{∗}h/mL in a patient; preferably, the compound represented by formula (I) or the pharmaceutically acceptable salt thereof provides an AUC of 3400-4400 ng^{∗}h/mL in a patient; preferably, the compound represented by formula (I) or the pharmaceutically acceptable salt thereof provides an AUC of 3600-4200 ng^{∗}h/mL in a patient.

The method for increasing the AUC of the compound represented by formula (I) or the pharmaceutically acceptable salt thereof in the patient with anastrozole provided by the present disclosure, optionally simultaneously increases the Cₘₐₓ of the compound represented by formula (I) or the pharmaceutically acceptable salt thereof in the patient.

In the optional embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof alone, the Cₘₐₓ ratio of the compound represented by formula (I) or the pharmaceutically acceptable salt thereof in a patient is more than 1:1, optionally, the Cₘₐₓ ratio is selected from 1 to 10:1; optionally, the Cₘₐₓ ratio is selected from 1 to 5:1; optionally, the Cₘₐₓ ratio is selected from 1 to 4:1; optionally, the Cₘₐₓ ratio is selected from 1 to 3:1; preferably, the Cₘₐₓ ratio is selected from 1 to 2:1; and preferably, the Cₘₐₓ ratio is selected from 1 to 1.5:1. The specific Cₘₐₓ ratio may be 1.02 :1, 1.04 :1, 1.06 :1, 1.08 :1, 1.10 :1, 1.12 :1, 1.14 :1, 1.16 :1, 1.18 :1, 1.20 :1, 1.22 :1, 1.24 :1, 1.26 :1, 1.28 :1, 1.30 :1, 1.32 :1, 1.34 :1, 1.36 :1, 1.38 :1, 1.40 :1, 1.42 :1, 1.44 :1, 1.46 :1, 1.48 :1, 1.50 :1, 1.52 :1, 1.54 :1, 1.56 :1, 1.58 :1, 1.60 :1, 1.62 :1, 1.64 :1, 1.66 :1, 1.68 :1, 1.70 :1, 1.72 :1, 1.74 :1, 1.76 :1, 1.78 :1, 1.80 :1, 1.82 :1, 1.84 :1, 1.86 :1, 1.88 :1, 1.90 :1, 1.92 :1, 1.94 :1, 1.96 :1, 1.98 :1, 2.00:1.

The method for increasing AUC of the compound represented by formula (I) or the pharmaceutically acceptable salt thereof in the patient with anastrozole provided by the present disclosure may comprise administering 1-500 mg/day of the compound represented by formula (I) or the pharmaceutically acceptable salt thereof, preferably 50-200 mg/day of the compound represented by formula (I) or the pharmaceutically acceptable salt thereof, more preferably 100-150 mg/day of the compound represented by formula (I) or the pharmaceutically acceptable salt thereof, and 0.1-50 mg/day anastrozole, preferably 1-25 mg/day anastrozole, most preferably 1-10 mg/day anastrozole.

In some optional embodiments, the amount of the compound represented by formula (I) or the pharmaceutically acceptable salt thereof administered daily is 50 mg, 75 mg, 100 mg, 125 mg, 150 mg, 175 mg, preferably 100 mg, 125 mg or 150mg.

In the optional embodiments, the administration frequency of the pharmaceutical composition of the compound represented by formula (I) or a pharmaceutically acceptable salt thereofis once a day, twice a day, preferably once a day.

In the optional embodiments, the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg.

In the optional embodiments, the administration frequency of anastrozole is once a day, twice a day, preferably once a day.

In the optional embodiments, the amount of the compound represented by formula (I) or the pharmaceutically acceptable salt thereof administered daily is 50 mg, 75 mg, 100 mg, 125 mg, 150 mg, 175 mg, and the administration frequency of the compound represented by formula (I) or the pharmaceutically acceptable salt thereof is once a day, twice a day, preferably once a day; the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg, and the administration frequency is once a day, twice a day, preferably once a day.

In the preferable embodiments, the amount of the compound represented by formula (I) or the pharmaceutically acceptable salt thereof administered daily is 100 mg, 125 mg, 150 mg, and the administration frequency is once a day; the daily dose of anastrozole is 1.0 mg, and the administration frequency is once a day.

In the preferable embodiments, the amount of the compound represented by formula (I) or the pharmaceutically acceptable salt thereof administered daily is 150 mg, and the administration frequency is once a day; the daily dose of anastrozole is 1.0 mg, and the administration frequency is once a day.

In the preferable embodiments, the amount of the compound represented by formula (I) or the pharmaceutically acceptable salt thereof administered daily is 175 mg, and the administration frequency is once a day; the daily dose of anastrozole is 1.0 mg, and the administration frequency is once a day.

In the particularly preferable embodiments, the pharmaceutically acceptable salt of the compound represented by formula (I) is hydroxyethyl sulfonate.

The method of increasing the AUC of the compound represented by formula (I) or the pharmaceutically acceptable salt thereof in the patient with anastrozole provided by the present disclosure, wherein the compound represented by formula (I) or the pharmaceutically acceptable salt thereof may be administered in the form of a pharmaceutical composition; specifically, the pharmaceutical composition of the compound represented by formula (I) or the pharmaceutically acceptable salt thereof in the present disclosure may further contain at least one pharmaceutically acceptable excipient, the excipient is selected from diluent, binder, disintegrant and lubricant.

Preferably, in the pharmaceutical composition containing the compound represented by formula (I) or the pharmaceutically acceptable salt thereof, the content of the diluent may be 1%-99% by weight, preferably 5%-95% by weight relative to the total weight of the pharmaceutical composition; the content of the lubricant may be 0.01%-25% by weight, preferably 0.1%-10% by weight relative to the total weight of the pharmaceutical composition.

In the specific embodiments, anastrozole is administered in the form of a pharmaceutical composition suitable for enteral administration or parenteral administration, such as tablets, capsules,suppositories, ampoules, injection solutions or injection suspensions. Anastrozole can be administered, for example, in a commercially available form (e.g., ARIMIDEX○, R).

On the other hand, the present disclosure provides a method for treating tumor diseases, which comprises administering a therapeutically effective amount of the compound represented by formula (I) or the pharmaceutically acceptable salt thereof and a therapeutically effective amount of anastrozole to a patient.

The method for treating tumor diseases provided by the present disclosure, which may comprise administering a therapeutically effective amount of the compound represented by formula (I) or the pharmaceutically acceptable salt thereof and a therapeutically effective amount of anastrozole to a patient, wherein anastrozole increases the AUC of the compound represented by formula (I) or the pharmaceutically acceptable salt thereof in a patient. In an optional embodiment, anastrozole reduces the hematological toxicity of the compound represented by formula (I), and the hematological toxicity may be selected from neutropenia, leukopenia and/or thrombocytopenia, preferably grade 3, 4 neutropenia and/or grade 3, 4 leukopenia.

On the other hand, the present disclosure provides a method for reducing adverse reactions of a patient, wherein, the adverse reaction is preferably hematological toxicity, and the hematological toxicity may be selected from neutropenia, leukopenia and/or thrombocytopenia, preferably grade 3, 4 neutropenia and/or grade 3, 4 leukopenia; the method may comprise administering to a patient a therapeutically effective amount of the compound represented by formula (I) or the pharmaceutically acceptable salt thereof and a therapeutically effective amount of anastrozole, the patient may be a patient with tumor diseases.

The tumor diseasesareselected from one or more of sarcoma, lymphoma, lung cancer, bronchial cancer, prostate cancer, pancreatic cancer, gastrointestinal cancer, colon cancer, rectal cancer, colorectal adenoma, thyroid cancer, liver cancer, intrahepatic cholangiocarcinoma, hepatocellular carcinoma, adrenal cancer, gastric cancer, gastric tumor, glioma, glioblastoma, endometrial cancer, melanoma, kidney cancer, renal pelvis cancer, bladder cancer, uterine body cancer, cervical cancer, vaginal cancer, ovarian cancer, multiple myeloma, esophageal cancer, leukemia, acute myeloid leukemia, chronic myeloid leukemia, lymphocytic leukemia, myeloid leukemia, brain tumor, brain cancer, oral and pharyngeal cancer, laryngeal cancer, small intestine cancer, non-Hodgkin's lymphoma, melanoma, colonic villous adenoma, neoplasm, epithelial cancer, breast cancer, basal cell carcinoma, squamous cell carcinoma, actinic keratosis, tumor disease, neck or head tumor, primary thrombocythemia, myeloid metaplasticmyelofibrosis, and macroglobulinemia, preferably breast cancer.

The breast cancer is preferably hormone receptor positive (HR+) and/or human epidermal growth factor receptor 2 negative (HER2-); more preferably locally advanced or metastatic breast cancer of the above phenotype.

In the optional embodiments, the method for treating tumor provided by the present disclosure may comprise administering 1-500 mg/day of the compound represented by formula (I) or the pharmaceutically acceptable salt thereof, preferably 50-200 mg/day of the compound represented by formula (I) or the pharmaceutically acceptable salt thereof, more preferably 100-150 mg/day of the compound represented by formula (I) or the pharmaceutically acceptable salt thereof, and 0.1-50 mg/day anastrozole, preferably 1-25 mg/day anastrozole, most preferably 1-10 mg/day anastrozole.

In the optional embodiments, the amount of the compound represented by formula (I) or the pharmaceutically acceptable salt thereof administered daily is 50 mg, 75 mg, 100 mg, 125 mg, 150 mg, 175 mg, preferably 100 mg, 125 mg or 150mg.

In the optional embodiments, the administration frequency of the compound represented by formula (I) or a pharmaceutically acceptable salt thereof is once a day, twice a day, preferably once a day.

In the optional embodiments, the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg.

In the optional embodiments, the administration frequency of anastrozole is once a day, twice a day, preferably once a day.

In the optional embodiments, the amount of the compound represented by formula (I) or the pharmaceutically acceptable salt thereof administered daily is 50 mg, 75 mg, 100 mg, 125 mg, 150 mg, 175 mg, and the administration frequency of the compound represented by formula (I) or the pharmaceutically acceptable salt thereof is once a day, twice a day, preferably once a day; the daily dose of anastrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg, and the administration frequency is once a day, twice a day, preferably once a day.

In the preferable embodiments, the amount of the compound represented by formula (I) or the pharmaceutically acceptable salt thereof administered daily is 100 mg, 125 mg, 150 mg, and the administration frequency is once a day; the daily dose of anastrozole is 1.0 mg, and the administration frequency is once a day.

In the preferable embodiments, the amount of the compound represented by formula (I) or the pharmaceutically acceptable salt thereof administered daily is 150 mg, and the administration frequency is once a day; the daily dose of anastrozole is 1.0 mg, and the administration frequency is once a day.

In the preferable embodiments, the amount of the compound represented by formula (I) or the pharmaceutically acceptable salt thereof administered daily is 175 mg, and the administration frequency is once a day; the daily dose of anastrozole is 1.0 mg, and the administration frequency is once a day.

The method for treating tumor provided by the present disclosure may take a 28-day dosing cycle, wherein the compound represented by formula (I) or the pharmaceutically acceptable salt thereof is administered from the first day to the 21th day, then the administration is stopped for 7 days, and anastrozole is administered every day in a 28-day treatment cycle.

The present disclosure provides a method for treating locally recurrent or metastatic breast cancer in a human patient, comprising administering orally to the patient an effective amount of the compound represented by formula (I) or the pharmaceutically acceptable salt thereof, and an effective amount of anastrozole, the patient is a postmenopausal female patient or premenopausal female patient or perimenopausal female patient or a premenopausal female undergone ovariectomy,

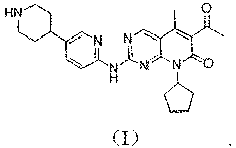

In the optional embodiments, in the method for treating locally recurrent or metastatic breast cancer provided by the present disclosure,every 28 days is a treatment cycle, wherein the compound represented by formula (I) or the pharmaceutically acceptable salt thereof is continuously administered from the first day to the 21th day, and anastrozole is administered every day in the 28-day treatment cycle.

In the optional embodiments, in the method for treating locally recurrent or metastatic breast cancer in human patients provided by the present disclosure, the patient is in the fasting state when the compound represented by formula (I) or the pharmaceutically acceptable salt thereof is administered.

In the optional embodiments, in the method for treating locally recurrent or metastatic breast cancer in the human patient provided by the present disclosure, the breast cancer is hormone receptor positive (HR+) and/or human epidermal growth factor receptor 2 negative (HER2-).

In the optional embodiments, in the method for treating locally recurrent or metastatic breast cancer in the human patient provided by the present disclosure, the daily dose of the compound represented by formula (I) or a pharmaceutically acceptable salt thereof is 100-150 mg.

In the optional embodiments, in the method for treating locally recurrent or metastatic breast cancer in the human patient provided by the present disclosure, the daily dose of the compound represented by formula (I) or a pharmaceutically acceptable salt thereof is 100 mg, 125 mg or 150 mg.

In the optional embodiments, in the method for treating locally recurrent or metastatic breast cancer in the human patient provided by the present disclosure, the daily dose of anastrozole is 0.1-10 mg.

In the optional embodiments, in the method for treating locally recurrent or metastatic breast cancer in the human patient provided by the present disclosure, the daily dose of anastrozole is 0.5 mg, 1 mg or 1.5mg.

In the optional embodiments, in the method for treating locally recurrent or metastatic breast cancer in the human patient provided by the present disclosure, the compound represented by formula (I) or a pharmaceutically acceptable salt thereof is administered in the morning.

In the optional embodiments, in the method for treating locally recurrent or metastatic breast cancer in the human patient provided by the present disclosure, the pharmaceutically acceptable salt of the compound represented by formula (I) is hydroxyethyl sulfonate.

On the other hand, the present disclosure provides a method for treating breast cancer, comprising administering 150 mg/day of the compound represented by formula (I) or the pharmaceutically acceptable salt thereof and 0.1-50 mg/day of anastrozole to a patient, monitoring adverse reactions (AE grade) of the patient; and if the patient has grade 3/4 AE, administering 125 mg/day or 100 mg/day of the compound represented by formula (I) or the pharmaceutically acceptable salt thereof.

Preferably, the method for treating breast cancer provided by the present disclosure, comprises administering 150 mg/day of the compound represented by formula (I) or the pharmaceutically acceptable salt thereof and 1 mg/day of anastrozole to a patient, monitoring adverse reactions (AE grade) of the patient; and if the patient has grade 3/4 AE, administering 125 mg/day or 100 mg/day of the compound represented by formula (I) or the pharmaceutically acceptable salt thereof.

Preferably, the method for treating breast cancer provided by the present disclosure, comprisesadministering 150 mg/day of the compound represented by formula (I) or the pharmaceutically acceptable salt thereof to a patient, administering orally once a day; administering 1 mg/day of anastrozole, administering orally once a day; monitoring adverse reactions (AE grade) of the patient; and if the patient has grade 3/4 AE, administering 125 mg/day or 100 mg/day of the compound represented by formula (I) or the pharmaceutically acceptable salt thereof to the patient.

Since anastrozole increases the AUC of the compound represented by formula (I) or the pharmaceutically acceptable salt thereof, the curative effect of the drug in the patient has a greater chance to be maintained after reducing the dose compared with the original dose.

On the other hand, the present disclosure provides a use of anastrozole in the preparation of a medicament increasing the AUC of the compound represented by formula (I) or the pharmaceutically acceptable salt thereof in a patient.

In the optional embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof alone, the AUC produced in the patient is increased by more than 18%, optionally, the AUC is increased by 20%-150%, optionally, the AUC is increased by 25%-100%; optionally, the AUC is increased by 30%-85%; optionally, the AUC is increased by 35%-80%; optionally, the AUC is increased by 40%-80%; preferably, the AUC is increased by 45%-80%; preferably, the AUC is increased by 50%-80%, preferably, the AUC is increased by 55%-80%; preferably, the AUC is increased by 60%-80%; specifically, the AUC of the compound represented by formula (I) or the pharmaceutically available salt thereof is increases by about 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130%, 131%, 132%, 133%, 134%, 135%, 136%, 137%, 138%, 139%, 140%, 141%, 142%, 143%, 144%, 145%, 146%, 147%, 148%, 149%, 150%.

In the optional embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof in combination with letrozole, the AUC ratio of the compound represented by formula (I) or the pharmaceutically available salt thereof in the patient is more than 1:1, optionally, the AUC ratio is selected from 1 to 10:1; optionally, the AUC ratio is selected from 1 to 5:1; optionally, the AUC ratio is selected from 1 to 4:1; optionally, the AUC ratio is selected from 1 to 3:1; preferably, the AUC ratio is selected from 1 to 2:1; preferably, the AUC ratio is selected from 1 to 1.5:1; specifically, the AUC ratio may be 1.01 :1, 1.02 :1, 1.03 :1, 1.04 :1, 1.05 :1, 1.06 :1, 1.07 :1, 1.08 :1, 1.09 :1, 1.10 :1, 1.11 :1, 1.12 :1, 1.13 :1, 1.14 :1, 1.15 :1, 1.16 :1, 1.17 :1, 1.18 :1, 1.19 :1, 1.20 :1, 1.21 :1, 1.22 :1, 1.23 :1, 1.24 :1, 1.25 :1, 1.26 :1, 1.27 :1, 1.28 :1, 1.29 :1, 1.30 :1, 1.31 :1, 1.32 :1, 1.33 :1, 1.34 :1, 1.35 :1, 1.36 :1, 1.37 :1, 1.38 :1, 1.39 :1, 1.40 :1, 1.41 :1, 1.42 :1, 1.43 :1, 1.44 :1, 1.45 :1, 1.46 :1, 1.47 :1, 1.48 :1, 1.49 :1, 1.50 :1, 1.51 :1, 1.52 :1, 1.53 :1, 1.54 :1, 1.55 :1, 1.56 :1, 1.57 :1, 1.58 :1, 1.59 :1, 1.60 :1, 1.61 :1, 1.62 :1, 1.63 :1, 1.64 :1, 1.65 :1, 1.66 :1, 1.67 :1, 1.68 :1, 1.69 :1, 1.70 :1, 1.71 :1, 1.72 :1, 1.73 :1, 1.74 :1, 1.75 :1, 1.76 :1, 1.77 :1, 1.78 :1, 1.79 :1, 1.80 :1, 1.81 :1, 1.82 :1, 1.83 :1, 1.84 :1, 1.85 :1, 1.86 :1, 1.87 :1, 1.88 :1, 1.89 :1, 1.90 :1, 1.91 :1, 1.92 :1, 1.93 :1, 1.94 :1, 1.95 :1, 1.96 :1, 1.97 :1, 1.98 :1, 1.99 :1, 2.00:1.

In the optional embodiments, by administering to the patient an effective amount of the compound represented by formula (I) or the pharmaceutically acceptable salt thereof and an effective amount of anastrozole, the compound represented by formula (I) or the pharmaceutically acceptable salt thereof provides an AUC of 2800-8000 ng^{∗}h/mL in a patient; optionally, the compound represented by formula (I) or the pharmaceutically acceptable salt thereof provides an AUC of 2800-7800 ng^{∗}h/mL in a patient; optionally, the compound represented by formula (I) or the pharmaceutically acceptable salt thereof provides an AUC of 3000-7600 ng^{∗}h/mL in a patient; optionally, the compound represented by formula (I) or the pharmaceutically acceptable salt thereof provides an AUC of 3000-7400 ng^{∗}h/mL in a patient; optionally, the compound represented by formula (I) or the pharmaceutically acceptable salt thereof provides an AUC of 3000-7200 ng^{∗}h/mL in a patient; optionally, the compound represented by formula (I) or the pharmaceutically acceptable salt thereof provides an AUC of 3000-7000 ng^{∗}h/mL in a patient; optionally, the compound represented by formula (I) or the pharmaceutically acceptable salt thereof provides an AUC of 3000-6800 ng^{∗}h/mL in a patient; optionally, the compound represented by formula (I) or the pharmaceutically acceptable salt thereof provides an AUC of 3000-6600 ng^{∗}h/mL in a patient; optionally, the compound represented by formula (I) or the pharmaceutically acceptable salt thereof provides an AUC of 3000-6400 ng^{∗}h/mL in a patient; optionally, the compound represented by formula (I) or the pharmaceutically acceptable salt thereof provides an AUC of 3000-6200 ng^{∗}h/mL in a patient; optionally, the compound represented by formula (I) or the pharmaceutically acceptable salt thereof provides an AUC of 3000-6000 ng^{∗}h/mL in a patient; optionally, the compound represented by formula (I) or the pharmaceutically acceptable salt thereof provides an AUC of 3000-5800 ng^{∗}h/mL in a patient; optionally, the compound represented by formula (I) or the pharmaceutically acceptable salt thereof provides an AUC of 3000-5600 ng^{∗}h/mL in a patient; optionally, the compound represented by formula (I) or the pharmaceutically acceptable salt thereof provides an AUC of 3000-5400 ng^{∗}h/mL in a patient; optionally, the compound represented by formula (I) or the pharmaceutically acceptable salt thereof provides an AUC of 3000-5200 ng^{∗}h/mL in a patient; optionally, the compound represented by formula (I) or the pharmaceutically acceptable salt thereof provides an AUC of 3000-5000 ng^{∗}h/mL in a patient; preferably, the compound represented by formula (I) or the pharmaceutically acceptable salt thereof provides an AUC of 3000-4800 ng^{∗}h/mL in a patient; preferably, the compound represented by formula (I) or the pharmaceutically acceptable salt thereof provides an AUC of 3200-4600 ng^{∗}h/mL in a patient; preferably, the compound represented by formula (I) or the pharmaceutically acceptable salt thereof provides an AUC of 3400-4400 ng^{∗}h/mL in a patient; preferably, the compound represented by formula (I) or the pharmaceutically acceptable salt thereof provides an AUC of 3600-4200 ng^{∗}h/mL in a patient.

In the use of anastrozole in the preparation of a medicament for increasing the AUC of the compound represented by formula (I) or the pharmaceutically acceptable salt thereof in the patient provided by the present disclosure, optionally anastrozole increases Cₘₐₓ of the compound represented by formula (I) or the pharmaceutically acceptable salt thereof in the patient.

In the optional embodiments, by administering to a patient an effective amount of the compound represented by formula (I) or the pharmaceutically acceptable salt thereof and an effective amount of anastrozole, relative to administering an equivalent dose of the compound represented by formula (I) or the pharmaceutically acceptable salt thereof alone, the Cₘₐₓ ratio of the compound represented by formula (I) or the pharmaceutically acceptable salt thereof in a patient is more than 1:1, optionally, the Cₘₐₓ ratio is selected from 1 to 10:1; optionally, the Cₘₐₓ ratio is selected from 1 to 5:1; optionally, the Cₘₐₓ ratio is selected from 1 to 4:1; optionally, the Cₘₐₓ ratio is selected from 1 to 3:1; preferably, the Cₘₐₓ ratio is selected from 1 to 2:1; and preferably, the Cmax ratio is selected from 1 to 1.5:1. The specific Cₘₐₓ ratio may be 1.02 :1, 1.04 :1, 1.06 :1, 1.08 :1, 1.10 :1, 1.12 :1, 1.14 :1, 1.16 :1, 1.18 :1, 1.20 :1, 1.22 :1, 1.24 :1, 1.26 :1, 1.28 :1, 1.30 :1, 1.32 :1, 1.34 :1, 1.36 :1, 1.38 :1, 1.40 :1, 1.42 :1, 1.44 :1, 1.46 :1, 1.48 :1, 1.50 :1, 1.52 :1, 1.54 :1, 1.56 :1, 1.58 :1, 1.60 :1, 1.62 :1, 1.64 :1, 1.66 :1, 1.68 :1, 1.70 :1, 1.72 :1, 1.74 :1, 1.76 :1, 1.78 :1, 1.80 :1, 1.82 :1, 1.84 :1, 1.86 :1, 1.88 :1, 1.90 :1, 1.92 :1, 1.94 :1, 1.96 :1, 1.98 :1, 2.00:1.

In the preferable embodiments, the dose of the compound represented by formula (I) or the pharmaceutically acceptable salt thereof is less than or equal to 175 mg, preferably less than or equal to 150 mg.

In the optional embodiments, the administration frequency of the compound represented by formula (I) or the pharmaceutically acceptable salt thereof is once a day or twice a day, and the daily dose of the compound represented by formula (I) or the pharmaceutically acceptable salt thereof is 1-500 mg, preferably 50-200 mg, more preferably 100-150 mg; the administration frequency of anastrozole is once a day or twice a day, and the daily dose is 0.1-50 mg, preferably 1-25 mg, most preferably 1-10 mg.

In the preferable embodiments, the administration frequency of the compound represented by formula (I) or the pharmaceutically acceptable salt thereof is once a day; the daily dose is 150 mg; and the administration frequency of anastrozole is once a day, the daily dose is 1 mg.

The present disclosure provides a method for increasing the AUC of the compound represented by formula (I) or the pharmaceutically acceptable salt thereof after administering, comprising administering an effective amount of anastrozole to a patient, 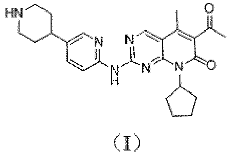 the patient has breast cancer, and the patient is in the fasting state when the compound represented by formula (I) or the pharmaceutically acceptable salt thereof is administered.

On the other hand, the present disclosure provides a method for treating tumor diseases, comprising administering to a patient a therapeutically effective amount of the compound represented by formula (I) or the pharmaceutically acceptable salt thereof and a therapeutically effective amount of at least one aromatic kinase inhibitor or estrogen receptor antagonist, the aromatic kinase inhibitor is selected from one or more of formestane, atamestane, anastrozole, fadrozole, letrozole, vorozole, exemestane and finrozole, preferably letrozole and/or anastrozole, the estrogen receptor antagonist is selected from one or more of tamoxifen, fulvestrant, preferably fulvestrant, wherein the compound represented by formula (I) or the pharmaceutically acceptable salt thereof is administered in the fasting state, the patient is preferably a human.

In the method for treating tumor diseases provided by the present disclosure, food, especially high-fat meal, may increase the exposure level of the compound represented by formula (I) or the pharmaceutically acceptable salt thereof; the therapeutic safety and effect of the drug is ensured by separating the administration and eating for a longer time, avoiding the pharmacokinetic effects of food on the compound represented by formula (I) or the pharmaceutically acceptable salt thereof.

In the optional embodiments, the aromatic kinase inhibitor is anastrozole, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of anastrazole is selected from 0.1-50 mg, preferably 1-25 mg, most preferably 1-10 mg, once a day, twice a day, preferably once a day, wherein the compound represented by formula (I) or the pharmaceutically acceptable salt thereof is administered in the fasting state.

In the optional embodiments, the aromatic kinase inhibitor is anastrozole, the dose of the compound represented by formula (I) or the pharmaceutically acceptable salt thereof is selected from 50 mg, 75 mg, 100 mg, 125 mg, 150 mg, 175 mg, preferably 100 mg, 125 mg or 150 mg, the administration frequency is once a day; and the dose of anastrazole is selected from 0.5 mg, 1.0 mg, 1.5 mg, 2.0 mg, 2.5 mg, 3.0 mg, 3.5 mg, 4.0 mg, 4.5 mg, 5.0 mg, preferably 1.0 mg, the administration frequency is once a day, wherein the compound represented by formula (I) or the pharmaceutically acceptable salt thereof is administered in the fasting state.

In the optional embodiments, the aromatic kinase inhibitor is anastrozole, the dose of the compound represented by formula (I) or the pharmaceutically acceptable salt thereof is 100 mg, 125 mg or 150 mg, the administration frequency is once a day; and the dose of anastrazole is 1.0 mg, the administration frequency is once a day, wherein the compound represented by formula (I) or the pharmaceutically acceptable salt thereof is administered in the fasting state.

In the optional embodiments, the aromatic kinase inhibitor is anastrozole, the dose of the compound represented by formula (I) or the pharmaceutically acceptable salt thereof is 100 mg, the administration frequency is once a day; and the dose of anastrazole is 1.0 mg, the administration frequency is once a day, wherein the compound represented by formula (I) or the pharmaceutically acceptable salt thereof is administered in the fasting state.

In the optional embodiments, the aromatic kinase inhibitor is anastrozole, the dose of the compound represented by formula (I) or the pharmaceutically acceptable salt thereof is 125 mg, the administration frequency is once a day; and the dose of anastrazole is 1.0 mg, the administration frequency is once a day, wherein the compound represented by formula (I) or the pharmaceutically acceptable salt thereof is administered in the fasting state.

In the optional embodiments, the aromatic kinase inhibitor is anastrozole, the dose of the compound represented by formula (I) or the pharmaceutically acceptable salt thereof is 150 mg, the administration frequency is once a day; and the dose of anastrazole is 1.0 mg, the administration frequency is once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of letrozole is selected from 1-100 mg, preferably 1-10 mg, most preferably 1-5 mg, the administration frequency is once a day, twice a day, preferably once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is selected from 50 mg, 75 mg, 100 mg, 125 mg, 150 mg, 175 mg, preferably 100 mg, 125 mg or 150 mg, the administration frequency is once a day; and the dose of letrozole is selected from 0.5 mg, 1.0 mg, 1.5 mg, 2.0 mg, 2.5 mg, 3.0 mg, 3.5 mg, 4.0 mg, 4.5 mg, 5.0 mg, preferably 2.5 mg, the administration frequency is once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is 100 mg, 125 mg or 150 mg, the administration frequency is once a day; and the dose of letrozole is 2.5 mg, the administration frequency is once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is 100 mg, the administration frequency is once a day; and the dose of letrozole is 2.5 mg, the administration frequency is once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is 125 mg, the administration frequency is once a day; and the dose of letrozole is 2.5 mg, the administration frequency is once a day, wherein the compound represented by formula (I) or the pharmaceutically acceptable salt thereof is administered in the fasting state.

In the optional embodiments, the aromatic kinase inhibitor is letrozole, the dose of the compound represented by formula (I) or the pharmaceutically acceptable salt thereof is 150 mg, the administration frequency is once a day; and the dose of letrozole is 2.5 mg, the administration frequency is once a day, wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of fulvestrant is selected from 1-2000 mg, preferably 10-1000 mg, most preferably 100-1000 mg, the administration frequency is once a week or once two weeks, wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is selected from 50 mg, 75 mg, 100 mg, 125 mg, 150 mg, 175 mg, preferably 100 mg, 125 mg or 150 mg, the administration frequency is once a day; and the dose of fulvestrant is selected from 200 mg, 300 mg, 400 mg, 500 mg, 600 mg, 700 mg, 800 mg, preferably 500 mg, the administration frequency is once a week or once two weeks, wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is selected from 100 mg, 125 mg or 150 mg, the administration frequency is once a day; and the dose of fulvestrant is 500 mg, the administration frequency is once a week or once two weeks , wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is 100 mg, the administration frequency is once a day; and the dose of fulvestrant is 500 mg, the administration frequency is once a week or once two weeks, wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is 125 mg, the administration frequency is once a day; and the dose of fulvestrant is 500 mg, the administration frequency is once a week or once two weeks, wherein the compound represented by formula (I) or the pharmaceutically acceptable salt thereof is administered in the fasting state.

In the optional embodiments, the estrogen receptor antagonist is fulvestrant, the dose of the compound represented by formula (I) or the pharmaceutically acceptable salt thereof is 150 mg, the administration frequency is once a day; and the dose of fulvestrant is 500 mg, the administration frequency is once a week or once two weeks, wherein the compound represented by formula (I) or the pharmaceutically acceptable salt thereof is administered in the fasting state. In the particularly preferable embodiments, the compound represented by formula (I) or the pharmaceutically acceptable salt thereof is administered in the fasting state in the morning.

In the present disclosure, the combination of the pharmaceutical composition containing the compound represented by formula (I) or the pharmaceutically acceptable salt thereof with anastrozole are superior to the combination of the compound represented by formula (I) or the pharmaceutically acceptable salt thereof with letrozolein the use in the preparation of a medicament for treating tumors, which can be manifested in a higher objective remission rate (ORR) or progression-free survival (PFS) or duration of remission (DoR) or disease control rate (DCR) or overall survival (OS), higher rates of complete remission (CR) and/or partial remission (PR) and/or stable disease (SD), and lower incidence of adverse events in the patient, which can also be used to demonstrate the effect of the present disclosure.

### Description of Terminology

"Treatment" includes the preventive and therapeutic treatment (including, but not limited to, remission, cure, symptom relief, symptom reduction) and delay of progression of tumor diseases or disorders.

"Pharmaceutical composition" refers to a mixture containing at least one therapeutic agent and at least one pharmaceutically acceptable excipient, the pharmaceutically acceptable excipient includes, but not limited to, diluent, binder, disintegrant, lubricant, the diluent includes, but not limited to, mannitol, dextrose microcrystalline cellulose, pregelatinized starch, calcium hydrogen phosphate, lactose, sorbitol; the binder may be selected from one or more of starch, sodium carboxymethylcellulose, polyvinylpyrrolidone, pregelatinized starch, hydroxypropylmethylcellulose and hydroxypropylcellulose; the disintegrant may be selected from one or more of cross-linked sodium carboxymethylcellulose, sodium carboxymethylstarch, low-substituted hydroxypropylcellulose and cross-linked povidone; the lubricant may be selected from one or more of talc, magnesium stearate, zinc stearate, and glycerylbehenate.

The pharmaceutical composition containing the compound represented by formula (I) or the pharmaceutically acceptable salt thereof and the pharmaceutical composition containing anastrozolein the present disclosure may also be administered in a parenteral form, the pharmaceutical composition may comprise pharmaceutically acceptable excipient, the excipient is selected from one or more of preservatives, stabilizers, solubilizers, emulsifiers, osmotic pressure regulatorss, buffers.

"Therapeutically effective" preferably refers to an amount of therapeutic agent that is therapeutically or more broadly also prophylactically effective against tumor progression.

The expression "in combination with" in the present invention is a mode of administration, which means that at least one dose of the the pharmaceutical composition containing the compound represented by formula (I) or the pharmaceutically acceptable salt thereof and anastrozole are administered within a certain period of time, wherein both of the two drugs exhibit pharmacological effects. The period of time can be within one administration cycle, preferably within 24 hours. The pharmaceutical composition containing the compound represented by formula (I) or the pharmaceutically acceptable salt thereof and anastrozole can be administered simultaneously or sequentially. This period of time includes the treatment in which the pharmaceutical composition containing the compound represented by formula (I) or the pharmaceutically acceptable salt thereof and anastrozole are administered via the same route or different routes.

"Cₘₐₓ" in the present disclosure refers to the peak concentration of the pharmacokinetic drug in the blood, which is an important index to evaluate the absorption rate of the drug and reflects the exposure characteristics of the drug in the body.

"AUC" refers to the area surrounded by the pharmacokinetic blood drug concentration curve on the time axis, which is an important index to evaluate the degree of drug absorption and reflects the exposure characteristics of the drug *in vivo.* Since the blood drug concentration in pharmacokinetic studies can only be observed up to a certain time point t, there are two ways to express AUC: AUC(₀₋ₜ) and AUC(_{0-∞}), the former is obtained according to trapezoidal area method, and the latter is calculated by the following formula: AUC(_{0-∞}) = AUC(₀₋ₜ)+terminal concentration/terminal elimination rate. The AUC described in the present disclosure is the mean AUC₀₋₂₄ of a patient after reaching a steady state through a single dose or multiple doses, preferably the mean AUC₀₋₂₄ of a patient after reaching a steady state through multiple doses (i.e., AUCₛₛ).

"High-fat meal" is a meal with high fat (providing about 50% of calories in food) and high calorie (about 800-1000 kcal), in which protein provides about 150 kcal, carbohydrate provides about 250 kcal and fat provides about 500-600 kcal.

"Fasting" means that the interval between administration and eating is at least 0.5 hours, or at least 1 hour, or at least 2 hours, or at least 3 hours, or at least 4 hours, or at least 5 hours, or at least 6 hours, or at least 7 hours, or at least 8 hours, specifically at least 0.5 hour before a meal, or at least 1 hour before a meal, or at least 1.5 hours before a meal, or at least 2 hours before a meal, or at least 2 hours after a meal, or at least 3 hours after a meal, at least 4 hours after a meal, preferably at least 0.5 hour before breakfast, or at least 1 hour before breakfast, or at least 1.5 hours before breakfast, or at least 2 hours before breakfast.

The criteria for determining the severity of adverse events in the present disclosure are based on the NCI-CTC AE version 4.03 of the grading standard for adverse drug events. In the event of an adverse event not listed in the NCI-CTC AE version 4.03 table, the following criteria is available for reference:

| **Grade** | **Clinical description of severity** |
|---|---|
| 1 | Mild; no clinical symptoms or mild clinical symptoms; only abnormalities in clinical or laboratory examination; no need for treatment |
| 2 | Moderate; less, local or non-invasive treatment is needed; the |

| | |
|---|---|
| | age-appropriate activities of daily living (ADL)using tools are limited, and the activities of daily living using tools refers to cooking, shopping, making phone calls, counting money, etc. |
| 3 | Severe illness or medically serious symptoms but temporarily not life-threatening; resulting in hospitalization or prolonged hospitalization; resulting in disability; limitations in self care ADL. Self care ADL refers to: bathing, dressing, undressing, eating, going to the bathroom, taking medication, etc., not bedridden |
| 4 | Life-threatening; urgent treatment is needed |
| 5 | Causing death |

### Brief description of the drawings

Figure 1. The mean (Mean±SD) blood drug concentration-time curve of hydroxyethyl sulfonate of the compound represented by formula (I) (PK concentration analysis of the population);
Figure 2. The median blood drug concentration-time curve of hydroxyethyl sulfonate of the compound represented by formula (I) (PK concentration analysis of the population);
Figure 3. Box and whisker diagram of the main PK parameters of hydroxyethyl sulfonate of the compound represented by formula (I) for the individual (PK parameter analysis of the population).

### Detailed description of the preferred embodiment

The present disclosure will be further described in conjunction with the embodiments, but these embodiments are not intended to limit the scope of the present disclosure.

Embodiment 1: Phase Ib/II clinical research of hydroxyethyl sulfonate of the compound represented by formula (I) (drug A)in combination with letrozole or anastrozole or fulvestrant for treating advanced breast cancer with positive hormone receptor and negative HER2

### Drug information

1) drug A, prepared according to the method provided by WO2016124067A, with the specification of 25 mg/tablet and 125 mg/ tablet;
2) Letrozole tablets, manufacturer: Jiangsu Hengrui Medicine Co.,Ltd., specification: 2.5 mg/tablet;
3) Anastrozole tablets (Rainide), manufacturer: AstraZeneca Pharmaceutical Co., Ltd., specification: 1 mg/tablet;
(4) Fulvestrant injection (Faslodex); manufacturer: Vetter Pharma-Fertigung GmbH &Co.KG; dosage form: injection; specification: 5 ml: 0.25 mg.

### Purpose of the experiment

The main research purposes of the first stage:
To evaluate the safety and tolerance of drug A in combination with letrozole, anastrozole or fulvestrant in the treatment of locally recurrent or metastatic breast cancer in the HR+, HER2-postmenopausal or premenopausal female.

The secondary research purposes of the first stage:
To evaluate the pharmacokinetic properties of drug A in combination with letrozole or anastrozole or fulvestrant in patients with advanced breast cancer;
To preliminarily evaluate the efficacy of drug A in combination with letrozole or anastrozole or fulvestrant in the treatment of advanced breast cancer.

The main research purposes of the second stage:
To evaluate the efficacy of drug A in combination with letrozole or anastrozole in the treatment of locally recurrent or metastatic breast cancer in the HR+, HER2-postmenopausal or premenopausal female.

The secondary research purposes of the second stage:
To evaluate the safety of drug A in combination with letrozole or anastrozole in patients with advanced breast cancer.

### Endpoint of the research

The main research endpoints of the first stage:
Incidence and severity of adverse events (AE) and serious adverse events (SAE) and abnormalities of laboratory examination.

The secondary research endpoints of the first stage:
Pharmacokinetic parameters of drug A in combination with letrozole or anastrozole or fulvestrant: AUC, Tₘₐₓ, t_{1/2} and Cₘₐₓ, etc.;
Objective Response Rate, ORR

The main research endpoints of the second stage:
Objective Response Rate, ORR

The secondary research endpoints of the second stage:
Progression Free Survival, PFS;
Duration of Response, DoR;
Disease Control Rate, DCR;
Incidence and severity of adverse events (AE) and serious adverse events (SAE) and abnormalities of laboratory examination.

### Experimental design

### The first stage

In order to effectively investigate the safety, pharmacokinetic properties and efficacy of drug A in combination with letrozole or anastrozole or fulvestrant, the following cohort (dose level) studies were planned:
Cohort 1: drug A 150 mg qd (d1-21) + letrozole 2.5 mg qd or anastrozole 1 mg qd;
cohort 2: drug A 125 mg qd (d1-21) + letrozole 2.5 mg qd or anastrozole 1 mg qd;
cohort 3: drug A 150 mg qd (d1-21) + fulvestrant 500 mg, intramuscular injection (first cycle: d1 and d15, followed by cycle d1);
cohort 4: drug A, dose to be determined qd (d1-21) + fulvestrant 500 mg, intramuscular injection (first cycle: d1 and d15, followed by cycle d1);

About 15 subjects were enrolled in each cohort (at least 10 subjects can be evaluated for tolerance and pharmacokinetics). The first cycle (28 days) of combined administration was used as the tolerance observation period, during which the dose group was considered tolerable if the proportion of subjects with clinically significant toxicity was ≤33% (i.e., no more than 3 out of 10 subjects who can be evaluated for tolerability had clinically significant toxicity). After the observation of the first cycle of cohort 1, the researcher and the sponsor jointly negotiated and decided the dose of cohort 2 according to the safety and tolerance results and the data of other relevant clinical studies. After the observation of the first cycle of cohort 3, the researcher and the sponsor jointly negotiated and decided the dose of cohort 4 according to the safety and tolerance results and the data of other relevant clinical studies.

Every 28 days was a treatment cycle: drug A was administered continuously in the first 3 weeks (from the first day to the 21th day), and rested (without administering) in the next 1 week (from the 22th day to the 28th day); letrozole or anastrozole was administered once a day. Fulvestrant was administered on the 1st day and the 15th day of the first cycle, followed by administration on the first day of each cycle.

### The second stage

According to the results of the first stage, the recommended dose of Phase II research (RP2D) was selected for further evaluation of efficacy and safety in patients with advanced breast cancer:
Drug A (RP2D, d1-21) + letrozole 2.5 mg qd or anastrozole 1 mg qd;

In this research, the screening period did not exceed 28 days, and subjects who were eligible after completing the screening examination and assessment entered the research treatment period (every 28 days was a treatment cycle), and research treatment and visit were carried out as specified in the scheme. Wherein, during the first 14 treatment cycles of the research treatment period, tumor imaging evaluation was conducted every 2 cycles (8 weeks ± 7 days);thereafter, tumor imaging evaluation was conductedevery 4 cycles (16 weeks ± 7 days). Subjects should visit the research center at the end of treatment/when withdrawing from the research to complete the corresponding safety inspection and imaging evaluation; and visit the research center 30 days after the last treatment to complete the corresponding safety assessment.

### Method of administration

In the first stage, subjects will be administered according to the cohort (dose level) they belong to as follows: cohort 1, drug A 150 mg, administered orally, once a day; cohort 2, drug A 125 mg, administered orally, once a day; cohort 3, drug A 150 mg, administered orally, once a day; cohort 4, the dose of drug A was to be determined, administered orally, once a day. Every 28 days was a treatment cycle, drug A (once a day) was administered continuously from the first day to the 21th day, and rested from the 22th day to the 28th day (drug A).

In the second stage, according to the results of the first stage, the recommended dose of Phase II research (RP2D) was used, drug A (RP2D), administered orally, once a day, every 28 days was a treatment cycle, drug A (once a day) was administered continuously from the first day to the 21th day, and rested (without administering) from the 22th day to the 28th day.

All subjects were administered drug A with letrozole or anastrozole or fulvestrantat the same time in the first stage. Letrozole 2.5 mg or anastrozole 1 mg, administered orally, once a day, continuously administered, every 28 days was a treatment cycle. It was recommended to administer drug A with letrozole or anastrozole orally at the same time with warm water at 8:00 every morning in the fasting state.

Subjects should continue to receive research treatment until disease progression, intolerable toxicity, termination of research treatment or withdrawal from the research voluntarily requested by the subject, or withdrawal from the research determined by the investigator.

### Entry criteria

All subjects must meet the following entry criteria to be eligible for this trial.
1. Female breast cancer patients diagnosed as HR+ and HER2- by pathological testing, with evidence of focal recurrence or metastasis, not suitable for surgical resection or radiation therapy for curative purposes, and with no clinical indications for chemotherapy.
2. Postmenopausal female aged 18 to75, menopause is defined as:
   a) previous bilateral oophorectomy, or
   b) age ≥ 60 years; or age < 60, natural postmenopausal state (defined as spontaneous cessation of regular menses for at least 12 consecutive months without other pathological or physiological causes), in addition, premenopausal or perimenopausal female patients may be enrolled provided they are willing to receive LHRH agonist therapy during the research period and have received LHRH agonist administration at least 14 days prior to enrollment in the research.
3. The score of physical status of the Eastern Cooperative Oncology Group (ECOG) is 0-1.
4. All acute toxic reactions caused by previous anti-tumor treatment or surgery are relieved to grade 0-1 (according to NCI CTCAE Version 4.03) or to the level specified by inclusion/exclusion criteria. Other toxicities such as alopecia that the investigator believes do not pose a safety risk to the patient are excluded.
5. Adequate organ and bone marrow function, defined as follows:
   a) Neutrophil count (ANC) ≥ 1,500/mm³ (1.5 × 10⁹/L);
   b) Platelet count (PLT) ≥ 100,000/mm³ (100 × 10⁹/L);
   c) Hemoglobin (Hb) ≥ 9 g/dL (90 g/L);
   d) Serum creatinine ≤ 1.5 times the upper limit of normal (ULN) or creatinine clearance rate ≥ 60 ml/min
   e) Total bilirubin (BIL) ≤ 1.5 times the upper limit of normal (ULN).
   f) Aspartate aminotransferase (AST/SGOT) or alanine aminotransferase (ALT/SGPT) levels ≤ 2.5 times the upper limit of normal (ULN), and patients with liver metastases should ≤ 5×ULN.
6. Female subjects of childbearing age must have a serum pregnancy test within 3 days before administering the research drug, and the result is negative, and they are willing to adopt a medically recognized high-efficiency contraceptive measure (such as intrauterine device, contraceptive or condom) during the research period and within 3 months after the last administration of the research drug.
7. Have given their consent and signed an informed consent form, and are willing and able to comply with planned visits, research treatment plans, laboratory tests and other trial procedures.
8. There are measurable lesions in accordance with RECIST 1.1 criteria.
9. Life expectancy > 12 weeks.

The subjects in cohort 1 and cohort 2 must meet all the following entry criteria to be eligible for this trial.

No prior systemic anticancer therapy for focal recurrent or metastatic disease.

The subjects in cohort 3 and cohort 4 must meet all the following entry criteria to be eligible for this trial:
1. Patients need to meet one of the following criteria:
   a) Recurrence or progression confirmed by imaging examination after 2 years after receiving adjuvant endocrine therapy, and no further endocrine therapy is received;
   b) recurrence or progression confirmed by imaging examination within 1 year after the completion of adjuvant endocrine therapy;
   c) recurrence or progression confirmed by imaging examination after 6 months after receiving first-line endocrine therapy for patients with metastatic diseases. Patients did not receive endocrine therapy or chemotherapy after the first line of metastatic disease.

Patients with recurrent and metastatic diseases are allowed to receive no more than first-line chemotherapy.

### Experimental results:

At present, 33 subjects had been included. Cohort 1: Drug A 150 mg, enrolled 17 cases, of which 13 cases were treated in combination with letrozole and 4 cases were treated in combination with anastrozole. Cohort 2: Drug A 125 mg, enrolled 16 cases, of which 15 cases were treated in combination with letrozole and 1 case was treated in combination with anastrozole. At least one efficacy evaluation was conducted in 32 subjects (cohort 1: 16 cases in the 150 mg dose group; cohort 2: 16 cases in the 125 mg dose group), and the safety evaluation was completed in 33 subjects at least within the first cycle.

Results of efficacy evaluation (as shown in Table 1 below): A total of 16 patients in the drug A 150 mg dose group underwent at least one efficacy evaluation, including 7 cases (44%) of PR, 1 case (6%) of PD and 8 cases (50%) of SD. A total of 16 patients in the drug A 125 mg dose group underwent at least one efficacy evaluation, including 5 cases (31%) of PR and 11 cases (69%) of SD. In cohorts 1 and 2, there were 5 subjects were treated in combination with anastrozole (4 cases in cohort 1; 1 case in cohort 2), and 4 of them were evaluated as PR at the first efficacy evaluation (2 months) after the first administration (as shown in Table 1 for details).

**Table 1. Phase Ib/II research efficacy of drug A**

| | **Drug A dose group** | **CR (N,%)** | **PR (N,%)** | **SD (N, %)** | **PD (N,%)** |
|---|---|---|---|---|---|
| Cohort 1 | 150 mg (N=16 cases) | 0 | 7 cases, 44% | 8 cases, 50% | 1 cases, 6% |
| | Combination with Anastrozole (N=4) | 0 | 3 cases, 75% | 1 cases, 25% | 0 |
| | Combination with letrozole (N=12) | 0 | 4 cases, 33% | 7 cases, 58% | 1 cases, 8% |
| Cohort 2 | 125 mg (N=16 cases) | 0 | 5 cases, 31% | 11 cases, 69% | 0 |
| | Combination with Anastrozole (N=1) | 0 | 1 case, 100% | 0 | 0 |
| | Combination with letrozole (N=15) | 0 | 4 cases, 27% | 11 cases, 73% | 0 |

The overall safety evaluation results: a total of 33 cases can be evaluated, including 17 cases in 150 mg dose group and 16 cases in 125 mg group. AE was reported for all subjects, most of subjects were grade 1-2. Grade 3-4 hematological AE mainly included leukopenia (9 cases, 27.3%) and neutropenia (20 cases, 60.6%). After a total of 57 subjects were enrolled in cohort 1 and 2, the group of compound A in combination with letrozole and the group of compound A in combination with anastrozole showed great differences in hematological toxicity. In 150 mg cohort, grade 3, 4 neutrophils decreased by 84.6% and 60.0% respectively in the two groups, grade 3, 4 leukocytes decreased by 38.5% and 20% respectivelyin the two groups, and platelets decreased by 23.1% and 5.0% respectively; in 125 mg cohort, grade 3, 4 neutrophils decreased by 73.3% and 44.4% respectively in the two groups, grade 3, 4 leukocytes decreased by 26.7% and 22.2% respectivelyin the two groups, and platelets decreased by 33.3% and 11.1% respectively; the data trends of the two cohorts were consistent, which indicated that compound A in combination with anastrozole was superior and safer in hematological toxicity than that in combination with letrozole.

The PK data for drug A in combination with letrozole and drug A in combination with anastrozole were compared with those for the single drug after multiple administration in the first administration cycle, and the results are shown in Table 2 and Table 3.

**Table 2.**

| Multiple administration (D21) | | Single-drug research | | Combination with letrozolegroup | | Combination with anastrozole group | |
|---|---|---|---|---|---|---|---|
| | | 125 mg group | 150 mg group | 125 mg group | 150 mg group | 125 mg group | 150 mg group |
| | | N=8 | N=8 | N=13 | N=12 | N=1 | N=4 |
| Cₘₐₓ | (ng/ml) | 121 (25.5) | 126 (26.2) | 138 (42.6) | 148 (33.4) | 147 | 192 (15.1) |
| AUCₛₛ | (h^{∗}ng/ml) | 2110 (29.5) | 2230 (21.5) | 2229 (36.8) | 2614 (34.5) | 2146 | 3686 (16.2) |
| t_{1/2z} | (h) | 36.2 (57.3) | 30.8 (24.7) | 54.5 (17.9) | 50.4 (17.5) | 46.5 | 46.0 (19.0) |
| CLₛₛ/F | (L/h) | 59.3 (29.2) | 67.2 (26.8) | 56.1 (36.8) | 57.4 (34.5) | 58.2 | 40.7 (16.2) |
| Racc_Cₘₐₓ | | 2.74 | 2.01 | 2.09 | 1.93 | 1.71 | 2.10 |
| Racc_AUC | | 3.53 | 2.71 | 3.12 | 2.77 | 2.35 | 3.06 |

**Table 3.**

| | | Combination with anastrozole/single drug (ratio) | | Combination with anastrozole/Combination with letrozole (ratio) | |
|---|---|---|---|---|---|
| Dose of drug A | Cycle | Cₘₐₓ ratio | AUC ratio | Cₘₐₓ ratio | AUC ratio |
| 125 mg | C1D1 | 1.15 | 1.47 | 1.31 | 1.28 |
| | C1D21 | 1.21 | 1.02 | 1.07 | 0.96 |
| 150 mg | C1D1 | 1.44 | 1.49 | 1.20 | 1.28 |
| | C1D21 | 1.52 | 1.65 | 1.30 | 1.41 |

| | | | | | |
|---|---|---|---|---|---|
| Note: Cₘₐₓ and AUC are both geometric mean value(CV%). | | | | | |

As shown in Table 2, the exposure levels of drug A in the 150 mg dose group were: combination with anastrozole>combination with letrozole> single drug.

As shown in Table 3, at the 150 mg dose, the AUC of the anastrozole group was 1.65 times higher than that of the single-drug research, and the AUC of the anastrozole group was 1.41 times higher than that of the letrozole group.

Wherein, in the 150 mg dose group of drug A in combination with anastrozole, one patient had grade 4 AE, and the patient maintained PR status after the dose was lowered to 125 mg in the third administration cycle.

In the later period, 9 patients were further enrolled in cohort 1, including 1 case treated in combination with letrozole and 8 cases treated in combination with anastrozole, and 5 patients were further enrolled in cohort 2, including 2 cases treated in combination with letrozole and 3 cases treated in combination with anastrozole. The PK data for drug A in combination with letrozole and drug A in combination with anastrozole were compared with those of the single drug after multiple administration in the first administration cycle, and the results are shown in Table 4 and Table 5.

**Table 4.**

| Multiple administration (D21) | | Single-drug research | | Combination with letrozole group | | Combination with anastrozole group | |
|---|---|---|---|---|---|---|---|
| | | 125 mg group | 150 mg group | 125 mg group | 150 mg group | 125 mg group | 150 mg group |
| | | N=8 | N=8 | N=15 | N=13 | N=4 | N=12 |
| Cₘₐₓ | (ng/ml) | 121 (25.5) | 126 (26.2) | 137 (39.6) | 151 (32.6) | 124 (17.0) | 173 (21.3) |
| AUCₛₛ | (h^{∗}ng/ml) | 2110 (29.5) | 2230 (21.5) | 2264 (34.5) | 2643 (33.3) | 2006 (19.2) | 3075 (22.8) |
| t_{1/2z} | (h) | 36.2 (57.3) | 30.8 (24.7) | 53.7 (17.1) | 50.5 (16.8) | 47.2 (3.91) | 47.6 (20.8) |
| CLₛₛ/F | (L/h) | 59.3 (29.2) | 67.2 (26.8) | 55.2 (34.5) | 56.8 (33.3) | 62.3 (19.2) | 48.8 (22.8) |
| Racc Cₘₐₓ | | 2.74 | 2.01 | 2.17 | 1.95 | 1.79 | 2.22 |
| Racc AUC | | 3.53 | 2.71 | 3.16 | 2.77 | 2.57 | 3.27 |

**Table 5.**

| | | Combination with anastrozole/single drug (ratio) | | Combination with anastrozole/Combination with letrozole (ratio) | |
|---|---|---|---|---|---|
| Dose of drug A | Cycle | Cₘₐₓ ratio | AUC ratio | Cₘₐₓ ratio | AUC ratio |
| 125 mg | C1D1 | 1.52 | 1.26 | 1.10 | 1.09 |
| | C1D21 | 1.02 | 0.951 | 0.906 | 0.886 |
| 150 mg | C1D1 | 1.23 | 1.16 | 1.01 | 0.987 |
| | C1D21 | 1.37 | 1.38 | 1.15 | 1.16 |

As shown in Table 4, the exposure levels of drug A in the 150 mg dose group were: combination with anastrozole>combination with letrozole> single drug.

As shown in Table 5, at the 150 mg dose, the AUC of the anastrozole group was 1.38 times higher than that of the single-drug research, and the AUC of the anastrozole group was 1.16 times higher than that of the letrozole group.

Embodiment 2: Effects of high-fat diet on the pharmacokinetic of healthy Chinese adult subjects after oral administration of tablets of hydroxyethyl sulfonate of the compound represented by formula (I)

### 1) Test drug

Tablets of hydroxyethyl sulfonate of the compound represented by formula (I), with specifications of 25 mg/ tablet and 125 mg/ tablet.

### 2) Administration regimen

In this research, 24 healthy adult subjects were enrolled; a randomized, open, single-dose, two-cycle, two-sequence, and cross-controlled trial design was adopted; the 24 healthy subjects were randomly divided into two sequence groups, A and B, 12 subjects in each sequence group were administered in the sequence of fasting→postprandial or postprandial→fasting, and the washing period was 14 days.

A sequence group: A single dose of 150 mg of tablets of hydroxyethyl sulfonate of the compound represented by formula (I) were administered in the fasting state on the first day, and PK plasma samples were collected from 0 to 144 hours, and a single dose of 150 mg of tablets of hydroxyethyl sulfonate of the compound represented by formula (I) were administered postprandially on the 15th day, and PK plasma samples were collected from 0 to 144 hours.

B sequence group: A single dose of 150 mg of tablets ofhydroxyethyl sulfonate of the compound represented by formula (I) were administered postprandially on the first day, and PK plasma samples were collected from 0 to 144 hours, and a single dose of 150 mg of tablets of hydroxyethyl sulfonate of the compound represented by formula (I) were administered in the fasting state on the 15th day, and PK plasma samples were collected from 0 to 144 hours.

### First cycle:

Subjects were admitted to the Phase I clinical research center at D-1, where they were served with a standard dinner and then fasted overnight for at least 10 hours. On the morning of the day of administration (D 1), the A sequence group was administered with 150 mg of tablets of hydroxyethyl sulfonate of the compound represented by formula (I) with 240 mL warm water in the fasting state in the morning, and then blood samples were collected from 0 to 144 hours. In the B sequence group, a high-fat meal was eaten in the morning, at 30 minutes of eating (timed from the start of eating), 150 mg of tablets of hydroxyethyl sulfonate of the compound represented by formula (I) were administered with 240 mL of warm water, and then blood samples were collected from 0 to 144 hours. Drinking water was prohibited from 1 hour before administration to 1 hour after administration (except the water contained in high fat/high calorie meal), and drinking water was free at other times. Fasting for 4 hours after administration, standard diet was provided 4 hours after administration and about 10 hours after administration, respectively. 72 hours after administration, they can leave the phase I clinical research center.

### Second cycle:

After a 14-day washout period, subjects were admitted to the Phase I clinical research center at D14, where they were served witha standard dinner and then fasted overnight for at least 10 hours. On the day of administration (D15), the B sequence group was administeredwith 150 mg of tablets of hydroxyethyl sulfonate of the compound represented by formula (I) with 240mL warm water in the fasting state in the morning, and then blood samples were collected from 0 to 144 hours. In the A sequence group, a high-fat meal was eaten in the morning, at 30 minutes of eating (timed from the start of eating), 150 mg of tablets ofhydroxyethyl sulfonate of the compound represented by formula (I) were administered with 240 mL of water, and then blood samples were collected from 0 to 144 hours. Drinking water was prohibited from 1 hour before administration to 1 hour after administration (except the water contained in high fat/high calorie meal), and drinking water was free at other times. Fasting for 4 hours after administration, standard diet was provided 4 hours after administration and about 10 hours after administration, respectively. 72 hours after administration, they can leave the phase I clinical research center.

Breakfast was required to be with high fat (providing about 50% of calories in food) and high calorie (about 800-1000 kcal), in which protein provided about 150 kcal, carbohydrate provided about 250 kcal and fat provided about 500-600 kcal.

### 3) Experimental results

### 1.Analysis of blood drug concentration

Based on the PK concentration analysis of the population, descriptive statistics were performed on the drug concentrations of the subjects according to the sampling time specified in the scheme, divided by the state for administration (fasting and postprandial). Statistics include: number of subjects (n), number of BQL (n of BQL), arithmetic mean (Mean), standard deviation (SD), median (Median), minimum (Min), maximum (Max), coefficient of variation (%CV), geometric mean (GeoMean), log standard deviation (SD log) and geometric coefficient of variation (%CVb).

Based on the PK concentration analysis of the population, mean/median blood drug concentration-time curves (linear and semi-logarithmic) were plotted for subjects in different administering states (fasting and postprandial) according to the sampling times specified in the scheme.

Based on the PK concentration analysis of the population, individual blood drug concentration-time curves(linear and semi-logarithmic) were plotted for subjects in different administering states (fasting and postprandial) according to actual sampling time.

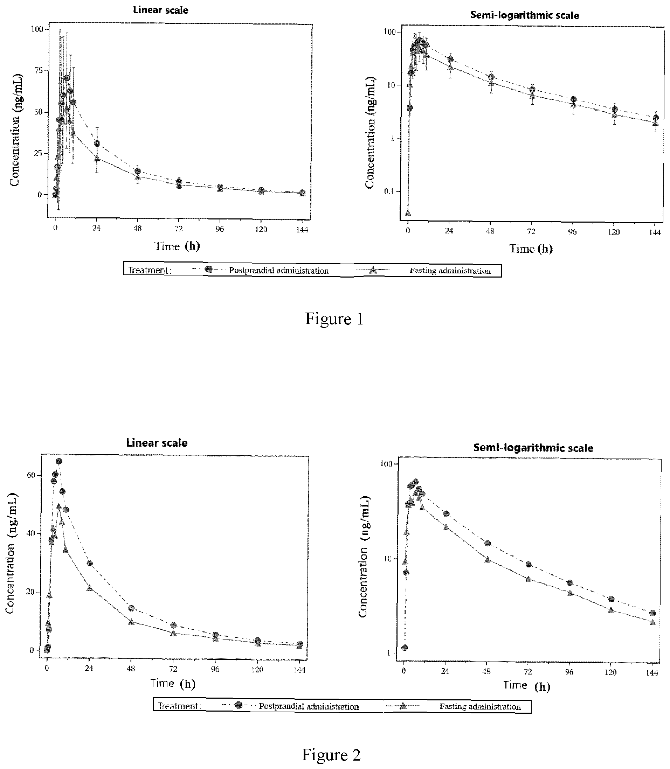

The mean (Mean±SD) blood drugconcentration-time curves for hydroxyethyl sulfonate of the compound represented by formula (I) was shown in Figure 1; the median blood drug concentration-time curves for hydroxyethyl sulfonate of the compound represented by formula (I) was shown in Figure 2.

### 2. PK parameter results

Based on the PK parameters analysis of the population, PK parameters were calculated based on the actual sampling time using the non-compartment model (NCA) of WinNonlin version 7.0, and the PK parameters were tabulated and descriptive statistics were performed using SAS version 9.4 software.

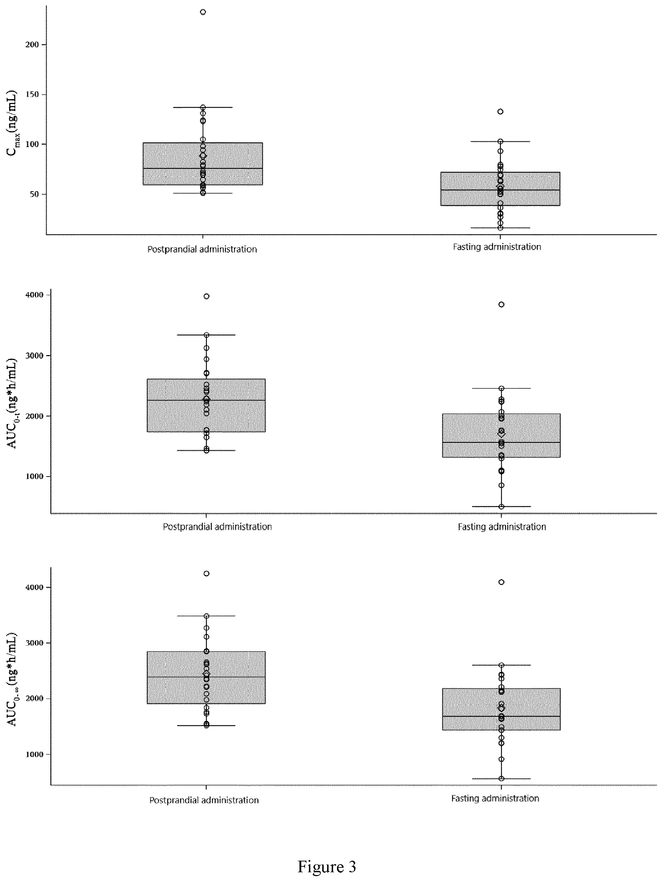

Box and whisker diagram of the main PK parameters (Cₘₐₓ, AUC₀₋ₜ and AUC_{0-∞}) for individual subjects at different administering states are shown in Figure 3.

The results of the descriptive statistical analysis of PK parameters for subjects at different administration states are shown in Table 6.

**Table 6. Summary table of PK parameters (PK parameters analysis of the population)**

| **PK parameters (unit)** | **Statistic** | **Fasting administration (N=24)** | **Postprandial administration (N=24)** |
|---|---|---|---|
| Tₘₐₓ(h) | Median(Min, Max) | 6.00 (1.00,8.00) | 5.00 (2.00, 10.0) |
| Cₘₐₓ(ng/mL) | Mean±SD | 58.2±27.1 | 88.2±40.2 |
| | Geomean(%CV_{b}) | 52.2 (53.2) | 81.8 (38.6) |
| AUC₀₋ₜ (h^{∗}ng/mL) | Mean±SD | 1708±668 | 2279±643 |
| | Geomean(%CV_{b}) | 1586 (42.4) | 2197 (28.0) |
| AUC_{0-∞} (h^{∗}ng/mL) | Mean±SD | 1841±697 | 2448±670 |
| | Geomean(%CV_{b}) | 1719 (40.6) | 2364 (27.4) |
| %AUCₑₓ(%) | Mean±SD | 7.72±2.83 | 7.00±2.64 |

| | Geomean(%CV_{b}) | 7.29 (35.0) | 6.62 (33.9) |
|---|---|---|---|
| λ_{z}(1/h) | Mean±SD | 0.0166±0.00270 | 0.0168±0.00260 |
| | Geomean(%CV_{b}) | 0.0164 (17.3 | 0.0166 (16.2 |
| t_{1/2} (h) | Mean±SD | 43.0±7.81 | 42.2±7.32 |
| | Geomean(%CV_{b}) | 42.4 (17.3) | 41.7 (16.2) |
| V_{z}/F (L) | Mean±SD | 6093±4108 | 3992±1215 |
| | Geomean(%CV_{b}) | 5334 (51.2) | 3813 (32.2) |
| **PK parameters (unit)** | **Statistic** | **Fasting administration (N=24)** | **Postprandial administration (N=24)** |
| CL/F (L/h) | Mean±SD | 94.5±45.0 | 65.7±17.5 |
| | Geomean(%CV_{b}) | 87.2 (40.6) | 63.5 (27.4) |

As can be seen from the figure, the distribution intervals of the Cₘₐₓ and AUC of the postprandial group and the fasting group were partially overlapped, but those of the postprandial group weregenerally higher than those of the fasting group, showing an obvious distribution difference, indicating that eating a high-fat meal had a tendency to increase the exposure level of hydroxyethyl sulfonate of the compound represented by formula (I).

As can be seen from the table, after oral administration of 150 mg of tablets of hydroxy ethyl sulfonate of the compound represented by formula (I) to the subjects in the fasting state and postprandially, the median Tₘₐₓ values were 6.00 h and 5.00 h respectively, indicating that the rate of absorption of hydroxyethyl sulfonate of the compound represented by formula (I) was less affected by eating a high-fat meal. The mean values of Cₘₐₓ were 58.2 ng/mL and 88.2 ng/mL, the mean values of AUC₀₋ₜ were 1708 h^{∗}ng/mL and 2279 h^{∗}ng/mL, and the mean values of AUC_{0-∞} were 1841 h^{∗}ng/mL and 2448 h^{∗}ng/mL after oral administration of 150 mg of tablets of hydroxyethyl sulfonate of the compound represented by formula (I) to the fasting and postprandial subjects, respectively; the Cₘₐₓ and AUC of the postprandial group were higher than those of the fasting group, indicating that eating a high-fat meal had a tendency to increase the absorption of hydroxyethyl sulfonate of the compound represented by formula (I). In addition, the average t_{1/2} of hydroxyethyl sulfonate of the compound represented by formula (I) in the fasting group was 43.0 h, and the average t_{1/2} of hydroxyethyl sulfonate of the compound represented by formula (I) in the postprandial group was 42.2 h, indicating that eating a high-fat meal has no significant effect on the elimination of hydroxyethyl sulfonate of the compound represented by formula (I).

### 3. Effect of high fat diet

Based on the PK parameters analysis of the population, the PK parameters (Cₘₐₓ, AUC₀₋ₜ and AUC_{0-∞}) of subjects at different administration state after natural logarithm transformation were analyzed using SAS version 9.4 software with mixed-effects model, and the results are shown in Table 7.

**Table 7 Effect of food on PK of hydroxyethyl sulfonate of the compound represented by formula (I) (PK parameters analysis of the population)**

| **Compare** | **PK parameters(unit)** | **Administration state** | **n** | **GeoLSM** | **Ratio (%)** | **90% CI of Ratio (%)** |
|---|---|---|---|---|---|---|
| Postprandial vs fasting | Cₘₐₓ(ng/mL) | Postprandial administration | 24 | 81.8190 | 156.85 | (138.42, 177.72) |
| | | In the fasting state | 24 | 52.1653 | | |
| | AUC₀₋ₜ(h^{∗}ng/mL) | Postprandial administration | 24 | 2197.4672138.57 | | (126.01,152.39) |
| | | In the fasting state | 24 | 1585.8079 | | |
| | AUC_{0-∞}(ng^{∗}h/mL) | Postprandial administration | 24 | 2363.9365 | 137.50 | (125.54, 150.59) |
| | | In the fasting state | 24 | 1719.2351 | | |

| | | | | | | |
|---|---|---|---|---|---|---|
| Note: GeoLSM is the geometrically adjusted mean value. | | | | | | |

As can be seen from the above table, after a single oral administration of 150 mg of tablets of hydroxyethyl sulfonate of the compound represented by formula (I) in subjects,the geometric mean ratios of Cₘₐₓ, AUC₀₋ₜ and AUC_{0-∞} were 156.85%, 138.57% and 137.50% in the postprandial group and the fasting group, respectively, and the exposure level of hydroxyethyl sulfonate of the compound represented by formula (I) increased by 37.5% to 56.9% after eating a high-fat meal compared with that in the fasting state; the 90% CI of the geometric mean ratios of Cₘₐₓ, AUC₀₋ₜ and AUC_{0-∞}were 138.42% to 177.72%, 126.01% to 152.39% and 125.54% to 150.59% in the postprandial group and the fasting group, respectively, none of which were within the range of 80.00% to 125.00% and all of which were higher than 125.00%. The above results indicate that high-fat diet has an effect on the exposure level of hydroxyethyl sulfonate of the compound represented by formula (I), and eating high-fat meal can increase the exposure level of hydroxyethylsulfonate of the compound represented by formula (I).

### 4) Experimental discussion and conclusion

After a single oral administration of 150 mg of tablets of hydroxyethyl sulfonate of the compound represented by formula (I), the blood drug concentration-time curves of the fasting group and the postprandial group showed the same trend, with time of peak in about 5~6 h, but the plasma exposure level of the postprandial group was higher than that of the fasting group. The semi-logarithmic drug-time curve showed a linear elimination of hydroxyethyl sulfonate of the compound represented by formula (I) inplasma after a single oral administration of tablets of hydroxyethyl sulfonate of the compound represented by formula (I).

In addition, after a single oral administration of 150 mg of tablets of hydroxyethyl sulfonate of the compound represented by formula (I), the geometric coefficients of variation of Cₘₐₓ, AUC₀₋ₜ and AUC_{0-∞} were 53.20%, 42.44% and 40.58% in the fasting group, respectively; the geometric coefficients of variation of Cₘₐₓ, AUC₀₋ₜ and AUC_{0-∞} were 38.57%, 28.00% and 27.38% in the postprandial group, respectively, and the PK parameters in the postprandial groupshowed a trend of decreasing variation compared with that in the fasting group.

High-fat diet has an effect on the exposure level of hydroxyethyl sulfonate of the compound represented by formula (I) in healthy subjects, and eating high-fat meal can increase the exposure level of hydroxyethyl sulfonate of the compound represented by formula (I). At 150 mg administered dose,the Cₘₐₓ, AUC₀₋ₜ and AUC_{0-∞} of hydroxyethylsulfonate of the compound represented by formula (I)in the postprandial group were increased by 56.9%, 38.6% and 37.5%, respectively, compared with those in the fasting group.

Although the above describes specific embodiments of the present invention, it should be understood by those skilled in the art that these are merely illustrative examples and that a variety of changes or modifications can be made to these embodiments without departing from the principles and substance of the present invention. Therefore, the scope of protection of the present disclosure is defined by the appended claims.

## Claims

1. A use of a pharmaceutical composition containing a compound represented by formula (I) or a pharmaceutically acceptable salt thereof in combination with anastrozole in the preparation of a medicament for treating tumor diseases,

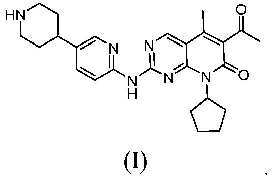

2. The use according to claim 1, wherein the tumor diseases are selected from one or more of sarcoma, lymphoma, lung cancer, bronchial cancer, prostate cancer, pancreatic cancer, gastrointestinal cancer, colon cancer, rectal cancer, colorectal adenoma, thyroid cancer, liver cancer, intrahepatic cholangiocarcinoma, hepatocellular carcinoma, adrenal cancer, gastric cancer, gastric tumor, glioma, glioblastoma, endometrial cancer, melanoma, kidney cancer, renal pelvis cancer, bladder cancer, uterine body cancer, cervical cancer, vaginal cancer, ovarian cancer, multiple myeloma, esophageal cancer, leukemia, acute myeloid leukemia, chronic myeloid leukemia, lymphocytic leukemia, myeloid leukemia, brain tumor, brain cancer, oral and pharyngeal cancer, laryngeal cancer, small intestine cancer, non-Hodgkin's lymphoma, melanoma, colonic villous adenoma, neoplasm, epithelial cancer, breast cancer, basal cell carcinoma, squamous cell carcinoma, actinic keratosis, tumor disease, neck or head tumor, primary thrombocythemia, myeloid metaplastic myelofibrosis, and macroglobulinemia, preferably breast cancer.

3. The use according to claim 2, wherein the breast cancer is hormone receptor positive (HR+) breast cancer.

4. The use according to claim 2, wherein the breast cancer is human epidermal growth factor receptor 2 negative (HER2-) breast cancer.

5. The use according to claim 3 or 4, wherein the breast cancer is locally advanced or metastatic breast cancer.

6. The use according to claim 1, the pharmaceutically acceptable salt of the compound represented by formula (I) is hydroxyethylsulfonate.

7. The use according to any one of claims 1-6, wherein the administration frequency of the pharmaceutical composition containing the compound represented by formula (I) or the pharmaceutically acceptable salt thereof is once a day or twice a day, and the daily dose of the compound represented by formula (I) or the pharmaceutically acceptable salt thereof is 1-500 mg, preferably 50-200 mg, more preferably 100-150 mg.

8. The use according to claim 7, wherein the administration frequency of the pharmaceutical composition containing the compound represented by formula (I) or the pharmaceutically acceptable salt thereof is once a day, the daily dose is 100 mg, 125 mg or 150 mg.

9. The use according to any one of claims 1 to 8, wherein the administration frequency of anastrozole is once a day or twice a day, the daily dose is 0.1-50 mg, preferably 1-25 mg, more preferably 1-10 mg.

10. The use according to claim 9, wherein the administration frequency of anastrozole is once a day, the daily dose is 1 mg.

11. The use according to any one of claims 7-10, wherein the administration frequency of the pharmaceutical composition containing the compound represented by formula (I) or the pharmaceutically acceptable salt thereof is once a day, the daily dose is 125 mg or 150 mg; the administration frequency of anastrozole is once a day, the daily dose is 1 mg.

12. The use according to any one of claims 7-11, wherein the pharmaceutical composition containing the compound represented by formula (I) or the pharmaceutically acceptable salt thereof and anastrozole are both administered orally.

13. The use according to any one of claims 1-12, wherein the pharmaceutical composition containing the compound represented by formula (I) or the pharmaceutically acceptable salt thereof further contain at least one pharmaceutically acceptable excipient, the excipient is selected from diluent, binder, disintegrant and lubricant.

14. The use according to claim 13, wherein, the content of the diluent is 1%-99% by weight, preferably 5%-95% by weight relative to the total weight of the pharmaceutical composition.

15. The use according to claim 13, wherein, the content of the lubricant is 0.01%-25% by weight, preferably 0.1%-10% by weight relative to the total weight of the pharmaceutical composition.

16. The use according to any one of claims 1-15, wherein anastrozole increases the AUC of the compound represented by formula (I) or the pharmaceutically acceptable salt thereof in a patient.

17. The use according to any one of claims 1-15, wherein anastrozole reduces the incidence of adverse reactions in a patient.

18. A use of anastrozole in the preparation of a medicament for increasing the AUC of a compound represented by formula (I) or a pharmaceutically acceptable salt thereof in a patient,

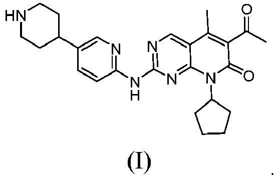

19. The use according to claim 18, wherein, relative to administering an equivalent dose of the compound represented by formula (I) or the pharmaceutically acceptable salt thereof alone, the AUC of the compound represented by formula (I) or the pharmaceutically acceptable salt thereof produced in the patient is increased by more than 18%, preferably, the AUC is increased by 20%-150%, more preferably, the AUC is increased by 25%-100%.

20. The use according to claim 18, wherein, relative to administering an equivalent dose of the compound represented by formula (I) or the pharmaceutically acceptable salt thereof in combination with letrozole, the AUC ratio of the compound represented by formula (I) or the pharmaceutically available salt thereof in the patient is more than 1:1, preferably, the AUC ratio is selected from 1 to 10:1; preferably, the AUC ratio is selected from 1 to 5:1.

21. The use according to claim 18, wherein, by administering to the patient an effective amount of the compound represented by formula (I) or the pharmaceutically acceptable salt thereof and an effective amount of anastrozole, the compound represented by formula (I) or the pharmaceutically acceptable salt thereof provides an AUC of 2800-8000 ng^{∗}h/mL in the patient; preferably, the compound represented by formula (I) or the pharmaceutically acceptable salt thereof provides an AUC of 3000-4800 ng^{∗}h/mL in the patient; preferably, the compound represented by formula (I) or the pharmaceutically acceptable salt thereof provides an AUC of 3200-4600 ng^{∗}h/mL in the patient.

22. The use according to any one of claims 19-21, wherein,the dose of the compound represented by formula (I) or the pharmaceutically acceptable salt thereof is less than or equal to 175 mg, preferably less than or equal to 150 mg.

23. The use according to claim 18, wherein, the administration frequency of the compound represented by formula (I) or the pharmaceutically acceptable salt thereof is once a day or twice a day, and the daily dose of the compound represented by formula (I) or the pharmaceutically acceptable salt thereof is 1-500 mg, preferably 50-200 mg, more preferably 100-150 mg; the administration frequency of anastrozole is once a day or twice a day, and the daily dose is 0.1-50 mg, preferably 1-25 mg, most preferably 1-10 mg.

24. The use according to claim 18, wherein the administration frequency of the pharmaceutical composition containing the compound represented by formula (I) or the pharmaceutically acceptable salt thereof is once a day, the daily dose is 150 mg; the administration frequency of anastrozole is once a day, the daily dose is 1 mg.

25. A method for treating tumor diseases, wherein, which comprises administering a therapeutically effective amount of the compound represented by formula (I) or the pharmaceutically acceptable salt thereof and a therapeutically effective amount of at least one aromatic kinase inhibitor or estrogen receptor antagonist to a patient, 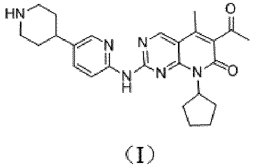 the aromatic kinase inhibitor is selected from one or more of formestane, atamestane, anastrozole, fadrozole, letrozole, vorozole, exemestane and finrozole, preferably letrozole and/or anastrozole, the estrogen receptor antagonist is selected from one or more of tamoxifen, fulvestrant, preferably fulvestrant, wherein the compound represented by formula (I) or the pharmaceutically acceptable salt thereof is administered in the fasting state.

26. The method according to claim 25, wherein, the aromatic kinase inhibitor is anastrozole, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of anastrazole is selected from 0.1-50 mg, preferably 1-25 mg, most preferably 1-10 mg, once a day, twice a day, preferably once a day.

27. The method according to claim 25, wherein, the aromatic kinase inhibitor is letrozole, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of letrozole is selected from 1-100 mg, preferably 1-10 mg, most preferably 1-5 mg, the administration frequency is once a day, twice a day, preferably once a day.

28. The method according to claim 25, wherein, the estrogen receptor antagonist is fulvestrant, the dose of the compound represented by formula (I) or the pharmaceutically acceptable salt thereof is 1-500 mg, preferably 50-200 mg, more preferably 100-150 mg, the administration frequency is once a day, twice a day, preferably once a day; and the dose of fulvestrant is selected from 1-2000 mg, preferably 10-1000 mg, most preferably 100-1000 mg, the administration frequency is once a week or once two weeks .

29. The method according to claim 25, wherein the tumor diseasesareselected from one or more of sarcoma, lymphoma, lung cancer, bronchial cancer, prostate cancer, pancreatic cancer, gastrointestinal cancer, colon cancer, rectal cancer, colorectal adenoma, thyroid cancer, liver cancer, intrahepatic cholangiocarcinoma, hepatocellular carcinoma, adrenal cancer, gastric cancer, gastric tumor, glioma, glioblastoma, endometrial cancer, melanoma, kidney cancer, renal pelvis cancer, bladder cancer, uterine body cancer, cervical cancer, vaginal cancer, ovarian cancer, multiple myeloma, esophageal cancer, leukemia, acute myeloid leukemia, chronic myeloid leukemia, lymphocytic leukemia, myeloid leukemia, brain tumor, brain cancer, oral and pharyngeal cancer, laryngeal cancer, small intestine cancer, non-Hodgkin's lymphoma, melanoma, colonic villous adenoma, neoplasm, epithelial cancer, breast cancer, basal cell carcinoma, squamous cell carcinoma, actinic keratosis, tumor disease, neck or head tumor, primary thrombocythemia, myeloid metaplastic myelofibrosis, and macroglobulinemia, preferably breast cancer.

30. The method according to any one of claims 25-29, wherein, the pharmaceutically acceptable salt of the compound represented by formula (I) is hydroxyethyl sulfonate.

31. The use according to any one of claims 1-17, wherein the patient with the tumor diseases is a human.

32. The use according to any one of claims 18-24, wherein the patient is a human.

33. The methodaccording to any one of claims 25-30, wherein the patient is a human.

34. A method for treating locally recurrent or metastatic breast cancer in a human patient, wherein, comprising administering orally to a patient an effective amount of the compound represented by formula (I) or the pharmaceutically acceptable salt thereof, and an effective amount of anastrozole, 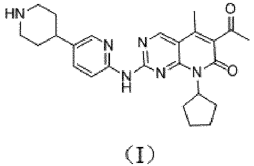 the patient is a postmenopausal female patient or premenopausal female patient or perimenopausal female patient or a premenopausal female undergone ovariectomy.

35. The method according to claim 34, wherein, every 28 days is a treatment cycle, wherein the compound represented by formula (I) or the pharmaceutically acceptable salt thereof is continuously administered from the first day to the 21st day, and anastrozole is administered every day in the 28-day treatment cycle.

36. The method according to claim 34, wherein, the patient is administered withthe compound represented by formula (I) or a pharmaceutically acceptable salt thereof in fasting state.

37. The method according to any one of claims 34- 36, wherein the breast cancer is hormone receptor positive (HR+) and/or human epidermal growth factor receptor 2 negative (HER2-) breast cancer.

38. The method according to claim 37, wherein the daily dose of the compound represented by formula (I) or the pharmaceutically acceptable salt thereof is 100-150 mg.

39. The method according to claim 38, wherein the daily dose of the compound represented by formula (I) or the pharmaceutically acceptable salt thereof is 100 mg, 125 mg or 150 mg.

40. The method according to claim 37, wherein the daily dose of anastrozole is 0.1-10 mg.

41. The method according to claim 40, wherein the daily dose of anastrozole is 0.5 mg, 1 mg or 1.5 mg.

42. The method according to any one of claims 34-41, wherein, the pharmaceutically acceptable salt of the compound represented by formula (I) is administered in the morning.

43. The method according to any one of claims 34-42, wherein, the pharmaceutically acceptable salt of the compound represented by formula (I) is hydroxyethyl sulfonate.

44. A method for increasing AUC of a compound represented by formula (I) or a pharmaceutically acceptable salt thereof after administration, which comprises administering an effective amount of the compound represented by formula (I) or the pharmaceutically acceptable salt thereof and anastrozole to a patient, 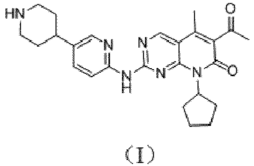 the patient has breast cancer, and the patient is in the fasting state when the compound represented by formula (I) or the pharmaceutically acceptable salt thereof is administered.
